(19) **Europäisches Patentamt / European Patent Office / Office européen des brevets**

(11) **EP 4 560 309 A1**

(12) **EUROPEAN PATENT APPLICATION**
published in accordance with Art. 153(4) EPC

(43) Date of publication:
**28.05.2025   Bulletin 2025/22**

(21) Application number: **23842657.1**

(22) Date of filing: **02.05.2023**

(51) International Patent Classification (IPC):
**G01N 27/62** (2021.01)

(52) Cooperative Patent Classification (CPC):
**G01N 27/62**

(86) International application number:
**PCT/JP2023/017129**

(87) International publication number:
**WO 2024/018725 (25.01.2024 Gazette 2024/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:   **21.07.2022   JP 2022116542**

(71) Applicant: **National Institute for Materials Science Tsukuba-shi, Ibaraki 305-0047 (JP)**

(72) Inventors:
• **NAITO Masanobu**
  **Tsukuba-shi, Ibaraki 305-0047 (JP)**
• **HIBI Yusuke**
  **Tsukuba-shi, Ibaraki 305-0047 (JP)**

(74) Representative: **J A Kemp LLP**
  **80 Turnmill Street**
  **London EC1M 5QU (GB)**

(54) **SEQUENCE ANALYZING METHOD, SEQUENCE ANALYZING DEVICE, POLYMERIZATION CONDITION PROPOSING DEVICE, AND AUTOMATIC SYNTHESIZING DEVICE**

(57)   According to a sequence analysis method for inferring the polyad content of a polymer, which polyad is formed by aligning a plurality of units, the method including determining the number $K$ of variants of the polyads; sequentially ionizing gas components generated by heating each sample of a reference sample and an inference target sample to acquire a data matrix including two-dimensional mass spectra; performing non-negative matrix factorization of the data matrix to factorize the data matrix into the product of a basis spectrum matrix and an intensity distribution matrix; performing non-negative matrix factorization of the intensity distribution matrix to factorize the matrix into the product of a matrix representing the mass proportion of a model polymer formed only from the polyad and a matrix representing a feature vector; defining the feature vector of the model polymer as an end member and determining a $K-1$ dimensional simplex including all of the feature vectors of samples; and inferring each polyad content ratio by the distance ratio between the end member and the feature vector of the inference target sample, a simple sequence analysis method for polymers is provided.

[FIG.1]

```
START
│
Determining the number K of variants of polyads.  — S1
│
Acquiring a data matrix.  — S2
│
├─────────────────────────┐
│                         │ YES
First NMF process.  — S3   │
│                         │
Second NMF process.  — S4  │
│                         │
Calculating model polymer  │
spectra (M spectra).  — S5 │
│                         │
Whether or not there is    │
an M spectrum which is not │
attributed to polyads. — S6│
│                         │
│ NO                      │
Determining a K-1          
dimensional simplex.  — S7
│
Inferring polyad
content ratios.  — S8
│
END
```

EP 4 560 309 A1

**Description**

Technical Field

The present invention relates to a sequence analysis method, a sequence analyzing device, a polymerization condition proposal device, and an automatic synthesizing device.

Background Art

As widely known in e.g. proteins and nucleic acid molecules, a sequence of units derived from monomers significantly affects the physical properties of polymer. For biopolymers such as proteins and nucleic acid molecules, many sequence analysis techniques have been proposed. As such techniques, Patent Literature 1 describes "a sequencing method for determining a sequence of a plurality of monomers forming a biopolymer by measuring a tunneling current flowing between a pair of electrodes, the method including a) a step of acquiring electric current value data by measuring an electric current value between the electrodes at prescribed time intervals, b) a step of selecting an analysis region including a dense region of signals from the electric current value data, c) a step of determining a minimum representative value having the smallest electric current value of a plurality of representative values and a maximum representative value having the maximum electric current value of a plurality of representative values in the analysis region, and d) a step of differentiating the type of monomer corresponding to the signal based on the minimum representative value and the maximum representative value."

Citation List

Patent Literature

Patent Literature 1: WO2019/208225

Summary of Invention

Technical Problem

As described above, sequence analysis methods for biopolymers, etc. have been established; however, for many polymers which are industrially used, a sequence analysis method has not been established yet. Therefore, a subject of the present invention is to provide a simple sequence analysis method for polymers. Another subject of the present invention is to provide a sequence analyzing device, a polymerization condition proposal device, and an automatic synthesizing device. Means of Solving Problem

As a result of diligent investigations to solve the above subjects, the present inventors found that the above subjects could be solved by the following constitution.

[1] A polymer sequence analysis method for inferring the polyad content of a polymer obtained by polymerizing monomers selected from a monomer set containing two or more types of monomers, which polyad is formed by aligning a plurality of units derived from the monomers, the method including determining the number K of variants of the polyads depending on the number of types of monomers contained in the monomer set, and the number of the units forming the polyads; sequentially ionizing gas components generated by heating each sample of a reference sample and an inference target sample, which are polymers formed from the monomers, to acquire a data matrix including two-dimensional mass spectra having m/z with respect to heating temperature; performing first NMF process by which the data matrix is subjected to non-negative matrix factorization to be factorized into the product of a matrix representing a normalized basis spectrum and an intensity distribution matrix; performing second NMF process by which the intensity distribution matrix in each of the samples is subjected to non-negative matrix factorization to be factorized into the product of a matrix representing a mass proportion of a model polymer formed only from the polyads in the sample, and a matrix representing a feature vector of the model polymer; defining the feature vector of the model polymer as an end member and determining a K-1 dimensional simplex including all of the feature vectors of the samples; and defining a distance between the K end members and a feature vector of the inference target sample by the Riemannian metric distance in consideration of non-orthogonality of the basis spectrum of first NMF process; and inferring each polyad content ratio of the inference target sample by the ratio of the distance.

[2] The sequence analysis method according to [1], wherein when the number K of variants is equal to or greater than 3, at least one of the feature vectors of the reference samples is present in each region external to a hypersphere inscribed in the K-1 dimensional simplex or the reference samples contain at least one of the end members.

[3] The sequence analysis method according to [1] or [2], further including after the second NMF process, reconstructing a spectrum of the model polymer by a matrix product of a matrix representing the feature vector of the model polymer and a matrix representing the basis spectrum to identify the polyad to which the spectrum of the model polymer is attributed.

[4] The sequence analysis method according to [3], wherein the identification is performed by comparing the sum of mass numbers of the units forming the polyad and m/z at peaks of the spectrum of the model polymer.

[5] The sequence analysis method according to [3] or [4], wherein when a spectrum of the model polymer not attributed to any of the polyads is present as a result of the identification, the number K of variants is changed and the first NMF process, the second NMF process and the identification are repeated.

[6] The sequence analysis method according to [5], wherein the change is to subtract a predetermined number from the number K of variants.

[7] The sequence analysis method according to [3] or [4], wherein when a spectrum of the model polymer which cannot be attributed to the polyads is present as a result of the identification, the reference samples are added and the data matrix acquisition, the first NMF process, the second NMF process and the identification are repeated.

[8] The sequence analysis method according to any one of [1] to [7], wherein when the number of the types is j and j is equal to or greater than 3, the number K of variants is determined by the formula: $K = {}_jC_3 + 3{}_jC_2 + {}_jC_1$.

[9] The sequence analysis method according to any one of [1] to [8], wherein the polymer includes a resist resin.

[10] The sequence analysis method according to any one of [1] to [9], wherein the reference sample and the inference target sample have different polymerization methods.

[11] A polymer sequence analyzing device for inferring the polyad content of a polymer obtained by polymerizing monomers selected from a monomer set containing two or more types of monomers, which polyad is formed by aligning a plurality of units derived from the monomers, the device including a mass spectrometer that sequentially ionizes gas components generated by heating a sample including a reference sample and an inference target sample, which are polymers formed from the monomers, and observes mass spectra continuously, and an information processing device that processes the observed mass spectra, wherein the information processing device includes a data matrix generating part that obtains a data matrix including two-dimensional mass spectra having m/z with respect to heating temperature; a variant number determining part that determines the number K of variants of the polyads depending on the number of types of monomers contained in the monomer set and the number of the units forming the polyads; a first NMF processing part that performs NMF process by which the data matrix is subjected to non-negative matrix factorization to be factorized into the product of a matrix representing a normalized basis spectrum and an intensity distribution matrix; a second NMF processing part that performs NMF process by which the intensity distribution matrix of each of the samples is subjected to non-negative matrix factorization to be factorized into the product of a matrix representing a mass proportion of a model polymer formed only from the polyad in the sample, and a matrix representing a feature vector of the model polymer and obtains the feature vector of the model polymer; a vector projection part that defines the feature vector of the model polymer as an end member and determines a K-1 dimensional simplex including all of the feature vectors of the samples; and a composition inference part that defines a distance between each of the K end members and a feature vector of the inference target sample by the Riemannian metric distance in consideration of non-orthogonality of the basis spectrum of first NMF process, and infers each polyad content ratio of the inference target sample by the ratio of the distance.

[12] The sequence analyzing device according to [11], further including a model polymer spectrum identification part that reconstructs a spectrum of the model polymer by the matrix product of a matrix representing the feature vector of the model polymer and a matrix representing the basis spectrum, and identifies the polyad to which the spectrum of the model polymer is attributed.

[13] The sequence analyzing device according to [12], wherein the identification is performed by comparing the sum of mass numbers of the units forming the polyad and m/z at peaks of the spectrum of the model polymer.

[14] The sequence analyzing device according to [12] or [13], wherein when a spectrum of the model polymer not attributed to any of the polyads is present as a result of the identification, the information processing device changes the number K of variants and repeats NMF process by the first NMF processing part, NMF process by the second NMF processing part, and identification by the model polymer spectrum identification part.

[15] A polymerization condition proposal device, further including the sequence analyzing device according to any one of [11] to [14], and a plan proposal part that has been subjected to machine learning using sequence analysis result by the sequence analyzing device and polymerization conditions of the inference target sample as training data, wherein the plan proposal part proposes a new polymerization condition to obtain, by comparing sequence analysis result and a predetermined target sequence, a polymer having the target sequence.

[16] An automatic synthesizing device, having the polymerization condition proposal device according to [15], and a synthesizing device for the polymer, wherein the synthesizing device has a feeding system for the monomers, a reaction tank that receives the monomers from the feeding system to allow the monomers to react, and a control device, wherein the control device controls at least one selected from the group consisting of the feeding system and the reaction tank based on a polymerization condition proposed by the polymerization condition proposal device to synthesize a new polymer.

Advantageous Effect of Invention

According to the present invention, it is possible to provide a simple sequence analysis method for polymers. Furthermore, according to the present invention, it is also possible to provide a sequence analyzing device, a polymerization condition proposal device and an automatic synthesizing device.

Brief Description of Drawings

Fig. 1 is a flowchart of a sequence analysis method according to one embodiment of the present invention;
Fig. 2 is M spectra calculated from respective matrices obtained by acquiring data matrices from samples synthesized using methyl methacrylate (M) and styrene (S) as monomers and performing first NMF process and second NMF process;
Fig. 3 is a conceptual view showing a K-1 dimensional simplex (two-dimensional simplex, a triangle) when K is 3;
Fig. 4 is a conceptual view showing a K-1 dimensional simplex (two-dimensional simplex, a triangle) when K is 3;
Fig. 5 is a hardware configuration view of a sequence analyzing device according to one embodiment of the present invention;
Fig. 6 is a functional block diagram of the sequence analyzing device;
Fig. 7 is a functional block diagram of a sequence analyzing device according to the second embodiment;
Fig. 8 is a functional block diagram of a polymerization condition proposal device according to one embodiment of the present invention;
Fig. 9 is a functional block diagram of an automatic synthesizing device according to one embodiment of the present invention;
Fig. 10 shows model polymer spectra of respective polyads obtained by calculation;
Fig. 11 shows calculation result of model polymer spectra when the number of types of monomers is 2 and the length of the polyads is 5;
Fig. 12 is a graph showing a relationship between polymerization time and conversion rate;
Figs. 13 are sequence analysis result and graphs showing a relationship between (A) the BBB content and (B) BBS content in terms of mass and conversion rate; and
Fig. 14 is sequence analysis result and a graph showing a relationship between the BBS content in terms of mass and conversion rate.

Description of Embodiments

The present invention will now be described in detail.
While constituting elements will be described below on the basis of representative embodiments of the present invention, the present invention is not restricted to these embodiments.
In the present description, a numerical value range expressed using "-" means a range including numerical values written before and after "-" as a lower limit value and an upper limit value.
The embodiments described below are examples of embodied technical ideas of the present invention, and the technical ideas of the present invention do not specify the material, shape, structure, arrangement, etc. of components to the embodiments described below. In addition, the drawings are schematic. Therefore, relationships between thickness and plane dimensions, ratios, etc. may be different from those of reality, and also dimension relationships and ratios between the drawings may be different from each other.

[Definition of Terms]

Terms used in the present description will be described. Terms which are not described below will be given a meaning generally understandable for persons in the art.
In the present description, a "monomer" means a compound (monomer) used to synthesize a polymer, a sample. Both a "reference sample" and an "inference target sample" are a polymer. The polymer is synthesized from one or two or more monomers selected from a monomer set consisting of a predetermined number of monomers.
In the present description, a "unit" is a part of a polymer structure and is derived from a monomer. In polyvinyl

chloride $(CH_2CHCl)_n$ synthesized by polymerization of vinyl chloride $(CH_2=CHCl)$, for example, vinyl chloride corresponds to the "monomer," polyvinyl chloride corresponds to the "polymer" and "$CH_2CHCl$" corresponds to the "unit."

In the present description, "polyads" mean a partial structure of a polymer formed by aligning a plurality of units with a finite number. Examples of polyads in a polymer synthesized from monomers A and B include diads such as AA, BB and AB (or BA); triads such as AAB; and the like as those which can be conceived on a combination basis.

The polyads are units for sequence analysis, and the sequence analysis in the present description means to infer the types of polyads included in an inference target sample and each polyad content in terms of mass.

In the above examples, "the number of units forming polyads" is 2 in e.g. AA, BB and AB (BA), and is 3 in e.g. AAA, AAB, BBA, BBB and ABA (BAB). In the description below, "the number of units forming polyads" may be simply referred to as "the length of polyads."

"The number of variants of polyads" means variations of combinations of units in the polyads. When the number of types of monomers contained in a monomer set is 3 (monomers A, B and C) and the number of units forming polyads (the length of polyads) is 3 (triad), for example, the number of variants of the triads is 13, AAA, BBB, CCC, AAC, AC{AC}, CCA, BBA, AB{BA}, AAB, BBC, BC{BC}, BCC and ABC. The "AC{AC}" means that "AC" is repeated. The repeats of "AC" can be represented as "ACA" and "CAC" as triads, but are represented as "AC{AC}" to be distinguished from "AAC{AAC}" and "CCA{CCA}," which are different sequences. The same applies to the others.

The number of variants of polyads is uniquely determined as the number of combinations which can be made depending on the length of polyads and the number of types of monomers contained in a monomer set. Some polyads which exist as theoretical combinations but cannot be actually generated, however, also exist depending on the types of monomers, polymerization forms, etc. When an alternating copolymer is not formed from monomers A and B, for example, "AB{AB}" is a polyad which exists as a theoretical combination but cannot be actually generated. Therefore, the number of variants of polyads is the number of combinations uniquely determined depending on the length of polyads and the number of types of monomers contained in a monomer set, or a number smaller than the above. In the present description, the number of variants of polyads can be represented as "K (a number of equal to or greater than 1)."

The "inference target sample" is a sample having a sequence to be inferred. The inference target sample is formed from one or two or more types of monomers, of two or more types of monomers contained in a sample set. Both the types of monomers used for synthesis and the amount thereof may be unknown. The inference target sample may be also a so-called homopolymer formed from one type of monomer.

In the present description, the "homopolymer" includes both a polymer actually formed only from one type of unit, and a polymer which is estimated (seemed) to be formed only from one type of unit on a mass spectrum. Specifically, even when a polymer is estimated to be formed only from one type of unit on a mass spectrum, but actually includes other units at equal to or smaller than the detection limit thereof, the polymer is treated as a "homopolymer" in the present description. The same applies to the reference sample about the treating.

The "reference sample" means a sample required in determining K end members in a K-1 dimensional simplex described later, and is a polymer synthesized from one or two or more types of monomers selected from the "sample set" as with the inference target sample.

The reference sample contains at least one or more types of polyads selected from K types of polyads. The types of polyads contained is not particularly restricted and may be 1 to K types. The reference samples may include a sample having the same composition as of the inference target sample. Specifically, while the inference target sample and the reference sample may be the same, the reference samples differ from each other.

The reference samples "differing" from each other mean that the types of units contained differ from each other, and at least one selected from the group consisting of sequences of units is different from the others.

The "end member" means a vector corresponding to a vertex of the K-1 dimensional simplex, and the end member corresponds to a feature vector of a polymer (model polymer) formed only from one of the K types of polyads.

[Sequence analysis method]

The sequence analysis method of the present invention is a sequence analysis method using as input the number K of variants determined depending on the number of types of monomers contained in the sample set and the number of units forming polyads, and two-dimensional mass spectra obtained from the reference samples and the inference target sample, and as output polyad content ratios in terms of mass of the inference target sample.

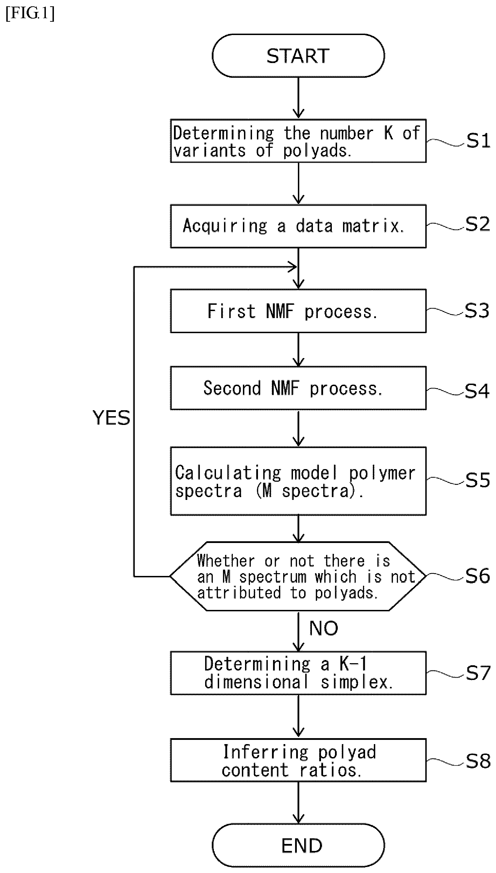

The sequence analysis method of the present invention will be described in detail with reference to drawings. Fig. 1 is a flowchart of an embodiment of the present invention.

First, the number K of variants of polyads (K is an integer of equal to or greater than 2) is determined depending on the number of types of monomers contained in a monomer set and the number of units forming the polyads as step S1.

The number of variants of polyads is a number which can be uniquely determined as the number of combinations which can be made depending on the length of the polyads and the number of types of monomers contained in the monomer set as an aspect.

The number of types of monomers is an integer of equal to or greater than 2, and the upper limit thereof is not particularly limited but preferably equal to or smaller than 10 as an aspect. When the number of types of monomers contained in the monomer set is 10, for example, the reference samples and the inference target sample are those synthesized from one or two or more types of the 10 types of monomers.

Specifically, the reference samples may be a (co)polymer obtained by adding one or two or more types of monomers selected from the sample set to a reaction container and polymerizing the monomers in various conditions (temperature and time). The inference target sample is only needed to be a sample synthesized from any one or two or more types of monomers contained in the monomer set.

The number of units forming polyads (the length of polyads) is equal to or greater than 2, and the upper limit thereof is not particularly restricted but preferably equal to or smaller than 10. Particularly, the length of polyads is preferably equal to or greater than 3, preferably equal to or smaller than 9, more preferably equal to or smaller than 7, and further preferably equal to or smaller than 5.

In the present sequence analysis method, the sequence of a polymer is inferred as polyad content ratios, and thus longer polyads get closer to uniquely defining the whole polymer chain. On the other hand, when the number of polyads is equal to or smaller than 10, increases in the number of variants of polyads and the number of end members are within a certain degree on a combination calculation basis, and thus the number of reference samples required does not easily increase.

When the length of polyads is equal to or greater than 3, the physical properties of a polymer are more easily predicted based on analysis result by the present sequence analysis method, and when the length is equal to or smaller than 5, the number of variations of the reference samples tends to become smaller and analysis tends to be easier.

When the number of types of monomers contained in the monomer set and the length of polyads are determined, the number K of variants of polyads can be uniquely determined. When the number of monomers is equal to or greater than 3 and the length of polyads is 3 (triad), for example, the number K can be calculated by $K = {}_jC_3 + 3_jC_2 + {}_jC_1$. When the number of monomers is 2, if the length of polyads is 2, 3, 4, 5, ..., K is 3, 5, 6, 9, ...

The length of polyads is uniquely determined from the number of theoretically possible combinations as described above; however, as described below, the feasible number can be smaller than the number, and K is preferably the number of theoretically possible combinations as described above or a number equal to or smaller than the above.

Next, gas components generated by heating each sample of the reference sample, which is a polymer formed from monomers, and the inference target sample are sequentially ionized, and a data matrix including two-dimensional mass spectra having m/z (originally written in italics, defined as a dimensionless quantity obtained by dividing ion mass by unified atomic mass unit and dividing the obtained value by the absolute value of ion charge number) with respect to heating temperature is acquired as step S2.

While a method of observing (acquiring) the mass spectrum is not particularly limited, a method of performing mass spectrometry without preparatory process on a specimen in an ambient condition is preferred. A mass spectrometer called "DART-MS" using an ion source called a "DART" (registered trademark, direct analysis in real time) ion source and a mass spectrometer instrument in combination is known as a device used in a method for the ionization and mass spectrometry described above.

The mass spectrometer instrument is not particularly limited but is preferably an instrument allowing precise mass spectrometer and may be any of types including quadripolar type and a time-of-flight (TOF) type.

While a specific condition for acquisition of the mass spectrum is not particularly restricted, according to a procedure given as a non-restrictive example, all the samples are sequentially heated at a temperature increasing rate of 50°C/min, and helium ions are injected at an interval of 50 shots/min to pyrolysis gas generated in a temperature range of 50-550°C to ionize the gas, thereby acquiring two-dimensional mass spectra having m/z along the horizontal axis and a temperature along the vertical axis.

The obtained two-dimensional mass spectra are stored for each sample and heating temperature as an aspect, and at least two or more of the two-dimensional mass spectra may be collectively converted into a data matrix.

In the present step, mass spectra are acquired continuously on the basis of each predetermined temperature increasing interval. While these mass spectra can be used as they are in generating the data matrix, they may be used after being averaged on the basis of each predetermined temperature increasing range. Averaging the mass spectra on the basis of each predetermined temperature increasing range and merging the mass spectra into one allow compression of a data volume. This temperature increasing range is approximately 10-30°C, for example.

In each spectrum, a peak intensity may be normalized. For example, this normalization can be realized by a method of normalizing peak intensities in such a manner that the sum of squares of the peak intensities becomes 1.

As a result, by making one measurement on a sample, it becomes possible to acquire a predetermined number of mass spectra at each heating temperature (or heating temperatures merged in each predetermined range) (this number differs depending on a way of the merging and may be 20, for example).

By storing each of these mass spectra in each row and the heating temperature in each column, a two-dimensional mass spectrum is acquired for each sample.

After the two-dimensional mass spectra are acquired for the respective samples in this way, at least two or more of these spectra are merged and converted to a data matrix X.

As long as the number of the two-dimensional mass spectra used in generating the data matrix X is equal to or greater than 2, it is not particularly limited. Meanwhile, it is preferable that two-dimensional mass spectra of all the samples (all the samples contained in the sample set) be used.

If one sample is subjected to measurement twice or more, some or all of two-dimensional mass spectra acquired by measurement twice or more may be used in generating the data matrix X.

Next, first NMF process is performed by which the data matrix is subjected to non-negative matrix factorization to be factorized into the product of a matrix including a normalized basis spectrum and an intensity distribution matrix as step S3.

The data matrix X is subjected to the first NMF process to be factorized into the product of an intensity distribution matrix A and a matrix S including a basis spectrum.

[Formula 1]

$$X \approx AS$$

Here, the data matrix X as input is represented by the following formula:

[Formula 2]

$$X \in \mathbb{R}_+^{NT \times D}$$

The intensity distribution matrix A and a matrix representing a basis spectrum (basis spectrum matrix) S as output are represented by the following formulas:

[Formula 3]

$$A \in \mathbb{R}_+^{NT \times M}$$

[Formula 4]

$$S \in \mathbb{R}_+^{M \times D}$$

Here, N is the number of samples, T is the number of temperature zone partitions, D is the number of channels, and M is the number of basis spectra. In the present description,

[Formula 5]

$$\mathbb{R}_+^{N \times M}, \quad \mathbb{R}^{N \times M}$$

The above is a non-negative or real-number N x M matrix.

Regarding the following matrix

[Formula 6]

$$X \in \mathbb{R}_+{}^{N \times M}$$

[Formula 7]

$$X_{n:} \in \mathbb{R}_+{}^{1 \times M}, X_{:m} \in \mathbb{R}_+{}^{N \times 1}, X_{nm} \in \mathbb{R}_+$$

represents an n-th row vector, an m-th row vector, and an (n, m)-th element, respectively. Furthermore, $X^T$ is a transposed matrix of X, and

[Formula 8]

$$\|X\|_F$$

represents a Frobenius norm. Furthermore,

[Formula 9]

$$\|X_{n:}\|_1 , \|X_{n:}\|_2$$

represent the following of an n-th row of X:

[Formula 10]

$$\ell_1\text{-norm}, \quad \ell_2\text{-norm}$$

Tr(X) represents a trace and diag(X) represents a diagonal matrix composed of a diagonal component. Furthermore, $1_N$ and $11_N$ represent an N-dimensional vector or an (N, N) matrix where all elements are 1. Moreover, $I_N$ represents an N-dimensional identity matrix.

Returning to the description of the first NMF process, the first NMF process in the present analysis method was developed so as to achieve better conformity with data interpretation of MS (mass spectrometry) by adding the following three points as main changes to ARD-SO-NMF suggested by Shiga, et al.

·Change 1: Variance-covariance matrix of Gaussian noise for each channel

[Formula 11]

$$R \in \mathbb{R}^{D \times D}$$

is estimated on the basis of a natural isotopic peak.

·Change 2: Application of soft orthogonal constraint between basis fragment spectra.

·Change 3: Merging of fragment spectra having similar intensity distributions (expansion of merging condition).

Regarding change 1, independent and identically distributed (i. i. d) Gaussian noise with a variance $\sigma^2$ is assumed for a while and then a variance-covariance matrix R is introduced in the middle. Assuming i. i. d in the noise, a probabilistic generative model for the data matrix X can be written as

[Formula 12]

$$p(X|A, S, \sigma^2) = \prod_{i=1}^{NT} \prod_{m=1}^{M} \frac{1}{\sqrt{2\pi\sigma^2}} exp \left\{-\frac{(X_{im} - [AS]_{im})^2}{2\sigma^2}\right\}$$

As the number M of basis spectra is unknown, automatic relevance determination (ARD) is introduced on the basis of sparseness of a distribution matrix A for allowing this number to be inferred automatically. First, an exponential distribution parametrized with $\lambda_m$ for each basis component is assumed as a prior distribution of A.

Specifically, with respect to the following:

[Formula 13]

$$\lambda = (\lambda_1, \dots, \lambda_M)^T \in \mathbb{R}^M$$

,

[Formula 14]

$$p(A_{im}|\lambda_m) = \frac{1}{\lambda_m} \exp\left(-\frac{A_{im}}{\lambda_m}\right) s.t. \lambda_m > 0, for\ i = 1, \dots, NT, m = 1, \dots, M,$$

$$p(A|\lambda) = \prod_{i=1}^{NT} \prod_{m=1}^{M} p(A_{im}|\lambda_m),$$

An entire probability model can be written as

[Formula 15]

$$p(X, A, S) = p(X|A, S, \sigma^2)p(A|\lambda)p(S)p(\lambda|a, b),$$

Here, p(S) is a uniform distribution on a hypersphere as follows:

[Formula 16]

$$\|\boldsymbol{S}_{n:}\|_2 = 1$$

Furthermore, p(λ|a, b) is an inverse gamma distribution parametrized with (a, b), which is specifically

[Formula 17]

$$p(\lambda|a,b) = \prod_{m=1}^{M} p(\lambda_m|a,b) = \frac{b^a}{\Gamma(a)} \lambda_m^{-(a+1)} \exp\left(-\frac{b}{\lambda_m}\right) for \ m = 1, \dots, M,$$

Here, a is a hyperparameter for adjusting sparseness, a = 1 + 10⁻⁶, and b can empirically be estimated from an expected value E ($A_{im}$) of $A_{im}$ and is related with
[Formula 18]

$$E(A_{im}) = \frac{b}{a-1} \tag{1}$$

This further has a relation with E($X_{id}$) as follows:

[Formula 19]

$$E(X_{id}) = \sum_{m=1}^{M} E(A_{im})E(S_{md}) = M \cdot E(A_{im})E(S_{md}),$$

By approximating E($X_{id}$) with an average $\mu_X$ of X

[Formula 20]

$$\mu_X = M \frac{b}{(a-1)\sqrt{D}}$$

Then, b is defined as follows:
[Formula 21]

$$b = \frac{\mu_X(a-1)\sqrt{D}}{M}, \tag{2}$$

Thus, a negative log likelihood function can be written as

[Formula 22]

$$L(X, A, S, \lambda) = -\log[p(X|A, S)p(A|\lambda)p(\lambda|a, b)]$$

$$= \frac{1}{2\sigma^2}\|X - AS\|_F^2 + \frac{DNT}{2}\log 2\pi\sigma^2 + (NT + a + 1)\sum_{m=1}^{M}\log\lambda_m$$

$$+ \sum_{m=1}^{M}\frac{1}{\lambda_m}\left(b + \sum_{i=1}^{NT}A_{im}\right) + M(\log\Gamma(a) - a\log b).$$

This is a downward-convex function with respect to $\lambda$. Thus, an update formula for $\lambda$ is obtained as

[Formula 23]

$$\frac{\partial L}{\partial \lambda} \equiv 0$$

,

specifically, as
[Formula 24]

$$\lambda_m = \frac{b + \sum_{i=1}^{NT}A_{im}}{NT + a + 1}, for\ m = 1, \ldots, M \tag{S3}$$

The deviation of the ARD-SO-NMF reported by Shiga, et al. has completely been followed so far.
The assumption of the i. i. d Gaussian distribution of the noise does not apply to MS data. A larger signal is known to be likely to have larger noise. Then, a noise distribution was determined as

[Formula 25]

$$\mathbf{E} \in \mathbb{R}^{NT \times D}$$

as a residual component of linear regression based on natural isotopic peaks. Specifically,
[Formula 26]

$$E_{:d} = X_{:d} - M^{(d)}\left(M^{(d)T}M^{(d)}\right)^{-1}M^{(d)T}X_{:d}, \, for \, d = 1, \dots, D, \quad (S4)$$

Here,

[Formula 27]

$$M^{(d)} = X_{:[d-30, d-20, d-10, d+10, d+20, d+30]}$$

is a channel [d-30, d-20, d-10, d+10, d+20, d+30] with respect to a channel d and is an isotropic peak of $\pm 3$ m/z of the channel d (note that channel spacing is 0.1 m/z). The variance-covariance matrix of each channel, which is given as

[Formula 28]

$$R \in \mathbb{R}^{D \times D}$$

, is determined as follows:

[Formula 29]

$$R = \frac{1}{NT}E^{T}E$$

Using this formula, the likelihood function is rewritten as

[Formula 30]

$$p(X|A, S, R) = \frac{1}{\sqrt{2\pi}^{DNT}\sqrt{|R|}^{NT}}\exp\{-Tr[(X - AS)R^{-1}(X - AS)^{T}]\}.$$

$$p(X|A, S, R) = \frac{1}{\sqrt{2\pi}^{DNT}\sqrt{|R|}^{NT}}\exp\{-Tr[(X - AS)R^{-1}(X - AS)^{T}]\}.$$

Note that R is a constant matrix. The total negative log likelihood function is defined as

[Formula 31]

$$L(X, A, S, \lambda) = -\log[p(X|A, S, R)p(A|\lambda)p(\lambda|a, b)]$$

$$= Tr[(X - AS)R^{-1}(X - AS)^T] + \frac{DNT}{2}log2\pi + \frac{NT}{2}log|R|$$

$$+ (NT + a + 1)\sum_{m=1}^{M} log\lambda_m + \sum_{m=1}^{M} \frac{1}{\lambda_m}\left(b + \sum_{i=1}^{NT} A_{im}\right)$$

$$+ M(log\Gamma(a) - alogb).$$

By substituting the update formula Eq. S3 for λ and making simplification by omitting a constant term,

[Formula 32]

$$L(X, A, S, \lambda) = Tr[(X - AS)R^{-1}(X - AS)^T] + (NT + a + 1)\sum_{m=1}^{M} log\lambda_m,$$

is obtained. This function is minimized with respect to A and S by using hierarchical alternating least square (HALS). For simplification, the following vector notation is used:

[Formula 33]

$$a_m \equiv A_{:m}, s_m \equiv S_{m:}^T, for \; m = 1, .., M.$$

According to the HALS, a residual X-AS is expressed as

[Formula 34]

$$X^{(m)} - a_m s_m^T \; (m = 1, ..., M)$$

.

Here,

[Formula 35]

$$X^{(m)} = X - AS + a_m s_m^T$$

Then, L(X, A, S, λ) can be written separately between components m as

[Formula 36]

$$L(X, a_m, s_m, \lambda_m(a_m))$$

$$= Tr\left[\left(X^{(m)} - a_m s_m{}^T\right)R^{-1}\left(X^{(m)} - a_m s_m{}^T\right)^T\right] + (NT + a + 1)\log\lambda_m,$$

$$for\ m = 1, \dots, M.$$

Soft orthogonal constraint on S can be incorporated as a penalty term as follows into an objective function L:

[Formula 37]

$$w_O \xi_m s_m^T s^{(m)}$$

Here,

[Formula 38]

$$s^{(m)} = \sum_{j \neq m}^M s_j$$

is established. This term represents non-orthogonality between an m-component and other components, and $\xi_m$ represents a Lagrange's undetermined multiplier applied if orthogonality is satisfied in a strict sense. This term is eased further using $w_O \in [0, 1]$. Thus, the objective function to be minimized is defined as

[Formula 39]

$$L(X, a_m, s_m, \lambda_m(a_m))$$

$$= Tr\left[\left(X^{(m)} - a_m s_m{}^T\right)R^{-1}\left(X^{(m)} - a_m s_m{}^T\right)^T\right] + (NT + a + 1)\log\lambda_m$$

$$+ w_O\, \xi_m s_m^T s^{(m)},$$

the gradients over $a_m$ and $s_m$ are as follows:

[Formula 40]

$$\frac{\partial L}{\partial \boldsymbol{a}_m} = \left(\boldsymbol{a}_m \boldsymbol{s}_m{}^T - \boldsymbol{X}^{(m)}\right)\boldsymbol{R}^{-1}\boldsymbol{s}_m + \frac{1}{\lambda_m}\boldsymbol{1}_{NT},$$

$$\frac{\partial L}{\partial \boldsymbol{s}_m} = \boldsymbol{R}^{-1}\left(\boldsymbol{s}_m \boldsymbol{a}_m^T - \boldsymbol{X}^{(m)}{}^T\right)\boldsymbol{a}_m + w_O\,\xi_m \boldsymbol{s}^{(m)},$$

and by setting these values zero, update formulas are written as

[Formula 41]

$$\boldsymbol{a}_m = \frac{\boldsymbol{X}^{(m)}\boldsymbol{R}^{-1}\boldsymbol{s}_m - \dfrac{1}{\lambda_m}\,\boldsymbol{1}_{NT}}{\boldsymbol{s}_m^T\boldsymbol{R}^{-1}\boldsymbol{s}_m}, \qquad\qquad (S5)$$

$$\boldsymbol{s}_m = \boldsymbol{X}^{(m)}{}^T\boldsymbol{a}_m - w_O\,\xi_m \boldsymbol{R}\boldsymbol{s}^{(m)}, \qquad\qquad (S6)$$

As $s_m$ is normalized in response to each update, a constant coefficient was omitted. Non-negative constraint was satisfied by being projected onto a non-negative quadrant in response to each update. As a specific example, the projection can be realized as

[Formula 42]

$$\boldsymbol{a}_m \leftarrow \frac{\boldsymbol{a}_m + |\boldsymbol{a}_m|}{2},$$

where,

[Formula 43]

$$|\boldsymbol{a}_m|$$

means a vector assuming an absolute value for each element. By multiplying Eq. S6 by

[Formula 44]

$$s^{(m)^T} R$$

from the left side and applying the following strict orthogonal condition and $w_o = 1$:

[Formula 45]

$$s^{(m)^T} s_m = 0$$

,

the undetermined multiplier $\xi_m$ is obtained as

[Formula]

$$-s^{(m)^T} X^{(m)^T} a_k + \xi_m s^{(m)^T} R s^{(m)} = 0,$$

$$\xi_m = \frac{s^{(m)^T} X^{(m)^T} a_k}{s^{(m)^T} R s^{(m)}} \tag{S7}$$

Using Eq. S1 to S7 given above, an algorithm 1 is suggested as follows:

[Formula 47]

<u>**Algorithm 1**: Pseudo-code of the first NMF</u>

**Input**: $\ell_1$-normalized data; $X \in \mathbb{R}_+^{NT \times D}$, orthogonal constraint weight; $w_O$, initial component number; $M$, iteration number; $itr$, margining threshold; $t \in [0.9, 0.99]$.

**Output**: spectra-wise fragment abundance matrix $A \in \mathbb{R}_+^{NT \times M}$, $M$-fragment spectra $S \in \mathbb{R}_+^{M \times D}$ with optimized $M$.

**Initialization**

initialize $A$ by Eq. S1

initialize $S$ by random selection of $M$-row vectors from $X$ and $\ell_2$-normalization.

calculate $b$ by Eq. S2

initialize $\lambda$ by Eq. S3

calculate $E$ by Eq. S4 and $R = \frac{1}{NT} E^T E$

**Repeat until convergence criteria are satisfied:**

**for** $m = 1, \ldots, M$:

    calculate $s^{(m)} \leftarrow \sum_{j \neq m}^M s_j$ and calculate $\xi_m$ by Eq. S7

    update $s_m$ by Eq. S6.

    project $s_m$ to the non-negative orthant

    $\ell_2$-normalize $s_m$

**for** $m = 1, \ldots, M$:

    update $a_m$ by Eq. S5

    project $a_m$ to the non-negative orthant


\# Merging very similar components

**for** $k = 1, \ldots, M - 1$:

    **for** $m = k, \ldots, M$:

      **if** $s_k^T s_m > t$:

        $a_k \leftarrow a_k + a_m, \; a_m \leftarrow 0$

      **if** $a_k^T a_m > t\|a_k\|\|a_m\|$:

        $s_k \leftarrow s_k + \frac{\|a_m\|_1}{\|a_k\|_1} s_m, \; s = \|s_k\|_2, \; s_k \leftarrow \frac{s_k}{s}$

        $a_k \leftarrow s a_k, a_m \leftarrow 0$

update $\lambda$ by Eq. S3

Here, each time A and S are updated, similar components are merged with each other. While merging of components having similar spectra with each other has been suggested by Shiga et al., components having similar intensity distributions are further subjected to the merging mentioned herein. By doing so, an isotopic peak, a fragment series ionized through addition of different ions, an oligomer peak series having different numbers of units, etc. can be merged into one component, making it possible to provide result with a higher degree of interpretability.

Furthermore, a noise component in the intensity distribution matrix is extracted through analysis on canonical correlation between the obtained basis spectrum matrix and the data matrix, and the intensity distribution matrix is corrected so as to reduce influence by the noise component, and thus the corrected intensity distribution matrix can be acquired.

The NMF is low-rank approximation of a data matrix. Thus, even if the component k is not actually present in an i-th spectrum, $C_{ik} > 0$ is established in a case where the presence of the component k provides better approximation in the sense of a least square. In many cases, such $C_{ik}$ is considerably small and often does not cause any problem in the NMF factorization.

In detecting a minor component, however, distinction is preferably made between $C_{jk} > 0$ actually present in tiny quantity in a j-th spectrum and $C_{ik} > 0$ as an NMF artifact, and 0 is preferably substituted into $C_{ik}$ as a ghost peak. The reason for this is that removing a spurious peak derived from the artifact from the NMF algorithm as one noise makes it possible to obtain inference result with higher precision.

Canonical correlation analysis can be employed as a method to solve the foregoing issue. The present method was named canonical correlation analysis (CCA) filter by the present inventors.

Conceptually, the CCA filter is to make sample-wise scanning to see whether each component in a basis spectrum output from the NMF is actually contained in original data, and to delete the component from the spectrum of a sample if a similar peak pattern is not observed in the original data. The CCA filter will now be described in detail.

Input is a basis spectrum matrix of the first NMF process output

[Formula 48]

$$S \in \mathbb{R}_+^{M \times D}$$

and a background spectrum:

[Formula 49]

$$X_{BG} \in \mathbb{R}_+^{T \times D}$$

Output is a list of polyads judged to be derived from background.

The m-component obtained by the first NMF process includes a component derived from background or a tramp material. As these might distort sequence analysis result, they are preferably removed from A or S. Assuming that an M'-component is judged to be a polyad derived from background by the CCA-filter,

[Formula 50]

$$A \in \mathbb{R}_+^{NT \times M}$$

and

[Formula 51]

$$S \in \mathbb{R}_+^{M \times D}$$

are defined as

[Formula 52]

$$A \in \mathbb{R}_+^{NT \times (M - M')}$$

and

[Formula 53]

$$S \in \mathbb{R}_+^{(M - M') \times D}$$

respectively. While the number of components after application of the CCA-filter is M - M' for simplification, M is still used consistently.

To apply the CCA-filter, a background spectrum

[Formula 54]

$$X_{BG} \in \mathbb{R}_+^{T \times D}$$

is required to be incorporated in a dataset and required to be used together with a sample spectrum for performing the first NMF. If the presence of any tramp material can be expected, a spectrum measured for the tramp material can be used as $X_{BG}$. According to the CCA-filter, components m = 1, ..., M are checked one by one to see whether their respective spectra $S_{m:}$ are contained in $X_{BG}$.

The first step is to partition S composed of an M-spectrum and subjected to:

[Formula 55]

$$\ell_2\text{-normalized}$$

into a spectrum set similar to $S_{m:}$:

[Formula 56]

$$Y \in \mathbb{R}_+^{M_{sim} \times D}$$

and into a spectrum set dissimilar to $S_{m:}$:

[Formula 57]

$$Z \in \mathbb{R}_+^{M_{dis} \times D}$$

.

This partitioning is performed in such a manner as to satisfy

[Formula 58]

$$S_{m:} Y_{m':}^{T} \geq t_1, for \ m' = 1, \dots, M_{sim},$$

$$S_{m:} Z_{m':}^{T} < t_1, for \ m' = 1, \dots, M_{dis},$$

. Here, $t_1 \in [0, 1]$ is a certain threshold and $t_1 = 0.2$ was consistently used in the present invention. Furthermore, as $S_{m:}$ is always contained in Y, $S_{m:}$ was stored in a first column of Y. Z is combined with $X_{BG}$ as

[Formula 59]

$$Z \leftarrow \begin{pmatrix} Z \\ X_{BG} \end{pmatrix},$$

In order to obtain an average zero, Y and Z were defined as

[Formula 60]

$$\overline{Y} = Y \left( I_D - \frac{1}{D} \mathbf{1} \mathbf{1}_D \right) \in \mathbb{R}^{M_{sim} \times D}, \qquad (S8)$$

$$\overline{Z} = Z \left( I_D - \frac{1}{D} \mathbf{1} \mathbf{1}_D \right) \in \mathbb{R}^{(M_{dis}+T) \times D}, \qquad (S9)$$

The CCA is applied to these two spectrum sets. The CCA generates a pair of spectra as similar as possible to each other through linear combination inside the two spectral sets. It is assumed that coefficients of linear combination of

[Formula 61]

$$\overline{Y}$$

and

[Formula 62]

$$\overline{Z}$$

are stored in a vector

[Formula 63]

$$u \in \mathbb{R}^{M_{sim}}$$

and in a vector

[Formula 64]

$$v \in \mathbb{R}^{M_{dis}+T}$$

.

Accordingly, the spectrum pairs can be written as

[Formula 65]

$$y \equiv u^T \overline{Y} \in \mathbb{R}^{1 \times D}$$

and

[Formula 66]

$$z \equiv v^T \overline{Z} \in \mathbb{R}^{1 \times D}$$

respectively. Similarity therebetween is evaluated using the correlation coefficient $\rho$ as

[Formula 67]

$$\rho = \frac{u^T V_{yz} v}{\sqrt{u^T V_{yy} u} \sqrt{v^T V_{zz} v}},$$

where

[Formula 68]

$$V_{YY} = \overline{Y}\overline{Y}^T/D, V_{ZZ} = \overline{Z}\overline{Z}^T/D, V_{YZ} = \overline{Y}\overline{Z}^T/D.$$

A problem setting by the CCA can be written as

[Formula 69]

$$(u^*, v^*) = arg\ max_{u,v}\ \rho.$$

A solution thereof, which is written as

[Formula 70]

$$\begin{pmatrix} u \\ v \end{pmatrix} \in \mathbb{R}^{M_{sim}+M_{dis}+T},$$

is provided as a solution of a generalized eigenvalue problem as

[Formula 71]

$$\begin{pmatrix} O & V_{yz} \\ V_{yz}^T & O \end{pmatrix}\begin{pmatrix} U \\ V \end{pmatrix} = \begin{pmatrix} V_{yy} & O \\ O & V_{zz} \end{pmatrix}\begin{pmatrix} U \\ V \end{pmatrix}\begin{pmatrix} \rho_1 & & \\ & \ddots & \\ & & \rho_{M_{sim}+M_{dis}+T} \end{pmatrix}, \rho_1 \geq \cdots \geq \rho_{M_{sim}+M_{dis}+T}, (S10)$$

, where

[Formula 72]

$$\begin{pmatrix} U \\ V \end{pmatrix}$$

is a matrix obtained by aligning the following characteristic vectors as column vectors in descending order of characteristic value:

[Formula 73]

$$\begin{pmatrix} u* \\ v* \end{pmatrix}$$

Each characteristic value

[Formula 74]

$$\left(\rho_1, \ldots, \rho_{M_{sim}+M_{dis}+T}\right)$$

is a coefficient of correlation between y and z generated through linear combination using corresponding (u\*, v\*) as a coefficient. Here, u\* corresponding to every characteristic value satisfying $\rho > t_2$ is extracted. If a first element corresponding to a coefficient of $S_{m:}$ is a large component largely contributing to u\*, specifically, if

[Formula 75]

$$\frac{|u_1|}{\|u^*\|_1} \geq t_3 \quad,$$

the component m is judged to be a component derived from background and removed from a system. Here, $t_2 \in [0.9, 0.99]$ and $t_3 \in [0, 1]$. These were summarized as follows as an algorithm 2:

[Formula 76]

<u>**Algorithm 2**</u>: CCA-filter

**Input**: $\ell_1$-normalized basis-spectra of the 1stNMF output; $S \in \mathbb{R}_+^{M \times D}$, a background spectrum; $X_{BG} \in \mathbb{R}_+^{T \times D}$, thresholds: $t_1 \in [0,1], t_2 \in [0,1], t_3 \in [0,1]$, in this study $(t_1, t_2, t_3) = (0.2, 0.9, 0.5)$ is consistently used.

**Output**: a list of the components judged as background components.

**for** $m = 1, \ldots, M$:

   **for** $m' = 1, \ldots, M$:

      classify $S_{m':}$ into $Y$ if $S_{m:}S_{m':}^T \geq t_1$ else into $Z$.

   $Z \leftarrow \begin{pmatrix} Z \\ X_{BG} \end{pmatrix}$

   calculate $\overline{Y}$ and $\overline{Z}$ by Eq. S8 and Eq. S9

   calculate $V_{YY} = \overline{Y}\overline{Y}^T/D, V_{ZZ} = \overline{Z}\overline{Z}^T/D, V_{YZ} = \overline{Y}\overline{Z}^T/D$

   obtain $U^* = (u^*_1, \ldots, u^*_{M_{sim}+M_{dis}+T})$ and $(\rho_1, \ldots, \rho_{M_{sim}+M_{dis}+T})$ by solving Eq. S10

   find $Q$ such that $\rho_Q \geq t_2$ and $\rho_{Q+1} < t_2$

   **for** $q = 1, \ldots, Q$:

      **if** $\frac{|U^*_{1q}|}{\|U^*_{:q}\|_1} \geq t_3$, add $m$ in the background-component list

  **return** the background-component list

After the background component is identified, a corresponding column vector in A and a corresponding row vector in S are deleted to be removed from the system.

After removal of the M'-component derived from background, the following is output from the CCA-filter:

[Formula 77]

$$A \in \mathbb{R}_+^{NT \times (M - M')}$$

(this is expressed as follows when M - M' is replaced with M for simplification:)

[Formula 78]

$$A \in \mathbb{R}_+^{NT \times M}$$

This is used for correcting an allocation intensity on the basis of a sample quantity and an internal reference peak. Then,

[Formula 79]

$$a_n = vec(A^{(n)})$$

, which is a one-dimensional vector generated from a submatrix section $A^{(n)}$ of A about a sample n, may be used as input in the second NMF as a feature vector of the sample n. Meanwhile, as an M-fragment temperature distribution is unnecessary information for composition analysis, all temperature zones may be added for each sample to represent M-fragment abundance (FA) for each sample, and the following representing this abundance may be used as input:

[Formula 80]

$$\tilde{A} \in \mathbb{R}_+^{N \times M}$$

Here, for simplification, the second NMF is performed on the following:

[Formula 81]

$$\tilde{A}$$

In the following section, non-negative least square (NNLS) fitting is performed frequently. Using an optimum non-negative coefficient as

[Formula 82]

$$x \in \mathbb{R}_+{}^n$$

and through column vector linear combination of a constant matrix as

[Formula 83]

$$\Phi \in \mathbb{R}^{m \times n},$$

the problem mentioned herein is to approximate:

[Formula 84]

$$y \in \mathbb{R}^m,$$

and is determined by the following:

[Formula 85]

$$x^* = \operatorname*{argmin}_{x} \|y - \Phi x\|^2, \, s.t. \, x \geq 0,$$

While this can be solved by a large number of optimization techniques including alternating direction multiplier methods (ADMM) (1), ADMM-NNLS developed by Fu. et al. (2) is used herein to solve this problem and a solution is represented as

[Formula 86]

$$x^* = NNLS(y, \Phi)$$

A non-negative coefficient vector $x_l^*$ ($l = 1, ..., L$) corresponding to an approximate vector set $Y = [y_1, ..., Y_L]$ can be calculated individually and is written as

[Formula 87]

$$x_l{}^* = NNLS(Y_{:l}, \Phi) \, for \, l = 1, .., L,$$

or in a matrix form, as

[Formula 88]

$$X^* = NNLS(Y, \Phi),$$

Here,

[Formula 89]

$$X^* = \left[ x_1{}^*, .., x_L{}^* \right]$$

.

A similar problem, which is a problem with a restraint condition that the total sum of coefficient vectors will be 1, is called fully constrained least square (FCLS), can also be solved using ADMM, and is written as

[Formula 90]

$$x_l{}^* = FCLS(Y_{:l}, \Phi) \ for \ l = 1, .., L,$$

or in a matrix notation, as

[Formula 91]

$$X^* = FCLS(Y, \Phi),$$

Next, second NMF process in step S4 will be described in detail. As shown in the following formula, the second NMF process is performed by which FA for each sample is subjected to matrix factorization to be factorized into the production of a matrix C representing the mass proportion of a model polymer in a sample and a matrix B representing a feature vector of the model polymer:

[Formula 92]

$$\widetilde{A} \approx CB$$

.

Input is FA for each sample:

[Formula 93]

$$\widetilde{A} \in \mathbb{R}_+{}^{N \times M}$$

,

a spectrum of each basis M-fragment:

[Formula 94]

$$S \in \mathbb{R}_+{}^{M \times D}$$

and the number K of variants. Output is a matrix representing the mass proportion of a model polymer in a sample:

[Formula 95]

$$C \in \mathbb{R}_+{}^{N \times K}$$

and a matrix representing a feature vector of the model polymer:

[Formula 96]

$$B \in \mathbb{R}_+{}^{K \times M}$$

Second NMF:

[Formula 97]

$$\widetilde{A} \approx CB$$

is evaluated in terms of its approximate residual as follows using Riemannian metrics:

[Formula 98]

$$D_G\left(\widetilde{A} \middle| CB\right)$$

Specifically,

[Formula 99]

$$D_G(\widetilde{A}|CB) = Tr\left[(\widetilde{A} - CB)G(\widetilde{A} - CB)^T\right],$$

where

[Formula 100]

$$G = SS^T \in \mathbb{R}_+^{M \times M}$$

By obtaining a lower triangular matrix as follows through Cholesky factorization G = LL^T

[Formula 101]

$$L \in \mathbb{R}^{M \times M},$$

the following formula can be written:

[Formula 102]

$$D_G(\widetilde{A}|CB) = Tr\left[(\widetilde{A} - CB)LL^T(\widetilde{A} - CB)^T\right] = Tr\left[(\widehat{A} - C\widehat{B})(\widehat{A} - C\widehat{B})^T\right] = \|\widehat{A} - C\widehat{B}\|_F^2,$$

Here,

[Formula 103]

$$\widehat{A} = \widetilde{A}L \in \mathbb{R}^{N \times M}, \widehat{B} = BL \in \mathbb{R}^{K \times M}$$

, which is equivalent to matrix factorization as follows:

[Formula 104]

$$\widehat{A} \approx C\widehat{B}$$

Defining a row vector as

[Formula 105]

$$\widehat{B},$$

a volume term of a simplex spanned by this row vector is written as

[Formula 106]

$$vol\left(\widehat{\boldsymbol{B}}\right), \widehat{\boldsymbol{B}}$$

and a non-orthogonality term between such row vectors is written as

[Formula 107]

$$nonorth\left(\widehat{\boldsymbol{B}}\right)$$

Then, using $\alpha > 0$ and $\beta \in [0, 1]$ as weights,
[Formula 108]

$$(\boldsymbol{C}^*, \widehat{\boldsymbol{B}}^*) = \underset{\boldsymbol{C},\widehat{\boldsymbol{B}}}{arg\ min} \frac{1}{2}\left\|\widehat{\boldsymbol{A}} - \boldsymbol{C}\widehat{\boldsymbol{B}}\right\|_F^2 + \frac{\alpha}{2}vol\left(\widehat{\boldsymbol{B}}\right) + \frac{\beta}{2}nonorth\left(\widehat{\boldsymbol{B}}\right), \qquad (S11)$$

$$s.t.\ \widehat{\boldsymbol{B}} = \boldsymbol{BL}, \boldsymbol{B} \geq 0, \boldsymbol{C} \geq 0, \boldsymbol{C}\mathbf{1}_K = \mathbf{1}_N.$$

On the basis of this formula,

[Formula 109]

$$\left(\boldsymbol{C}^*, \widehat{\boldsymbol{B}}^*\right)$$

is determined. Here, in order to provide robustness to an outlier (3), introduction of the following weighting matrix is suggested by Fu et al.:

[Formula 110]

$$w_n = \frac{p}{2}\left(\left\|\widehat{\boldsymbol{A}}_{n:} - \boldsymbol{C}_{n:}\widehat{\boldsymbol{B}}\right\|^2 + \varepsilon\right)^{\frac{p-2}{2}}$$

$$\boldsymbol{W} = diag(w_1, \dots w_N)$$

. Here, $p \in (0, 2]$, and $\varepsilon$ is a small regularization parameter. According to the present invention, $p = 1.5$ and $\varepsilon = 10^{-8}$ were consistently used. An optimization problem is written as

[Formula 111]

$$\min_{C,\widehat{B}} \frac{1}{2} Tr\left[W(\widehat{A} - C\widehat{B})(\widehat{A} - C\widehat{B})^T\right] + \frac{\alpha}{2} vol(\widehat{B}) + \frac{\beta}{2} nonorth(\widehat{B}),$$

$$s.t.\,\widehat{B} = BL, B \geq 0, C \geq 0, C1_K = 1_N.$$

Block coordinate descent (BCD) theory is employed to alternately optimize C and

[Formula 112]

$$\widehat{B}$$

Here, it is assumed that optimization is performed t times to obtain

[Formula 113]

$$\left(C^{(t)}, \widehat{B}^{(t)}\right)$$

Using vertex component analysis (VCA) (4) for initialization, K row vectors approximate to K-vertices of a simplex are selected from N-row vectors as

[Formula 114]

$$\widehat{A}$$

Then, the selected vectors are defined as

[Formula 115]

$$\widehat{B}^{(0)}$$

Regarding update of C based on the following:

[Formula 116]

$$\widehat{B}^{(t)}$$,

the update can be done simply by
[Formula 117]

$$C^{(t+1)^T} = FCLS\left(\widehat{A}^T, \widehat{B}^{(t)^T}\right). \qquad (S13)$$

Then, update based on C$^{(t)}$ from

[Formula 118]

$$\widehat{B}^{(t)}$$

to

[Formula 119]

$$\widehat{B}^{(t+1)}$$

will be considered. Here,

[Formula 120]

$$nonorth(\widehat{B}) = Tr\left(\Lambda(\widehat{B}\widehat{B}^T - diag(\widehat{B}\widehat{B}^T))\right),$$

and

[Formula 121]

$$\Lambda \in \mathbb{R}^{K \times K}$$

is an undetermined multiplier. Furthermore,

[Formula 122]

$$vol(\widehat{B}) = \log\left|\det\left(\widehat{B}\widehat{B}^T + \tau I_K\right)\right| = \log|\det(H)|,$$

where

[Formula 123]

$$H(\widehat{B}) = \widehat{B}\widehat{B}^T + \tau I_K, \tau$$

is a small regularization parameter, and $\tau = 10^{-8}$ was consistently used. Here,

[Formula 124]

$$vol\left(\widehat{\boldsymbol{B}}\right)$$

as a Majorizer function is introduced. Using a tangent inequality and on the basis of the following at previous timing:

[Formula 125]

$$\boldsymbol{H}^{(t)} = \boldsymbol{H}(\widehat{\boldsymbol{B}}^{(t)})$$,

[Formula 126]

$$\log|\det(\boldsymbol{H})| \leq \log\left|\det\left(\boldsymbol{H}^{(t)}\right)\right| + Tr\left[\left(\nabla_{\boldsymbol{H}^{(t)}}\log|\det(\boldsymbol{H})|\right)^{T}\left(\boldsymbol{H} - \boldsymbol{H}^{(t)}\right)\right],$$

is established. Here,

[Formula 127]

$$\nabla_{\boldsymbol{H}^{(t)}}\log|\det(\boldsymbol{H})|$$

is the slope of the following at $H^{(t)}$:

[Formula 128]

$$\log|\det(\boldsymbol{H})|$$.

Using

[Formula 129]

$$\nabla_{\boldsymbol{H}^{(t)}}\log|\det(\boldsymbol{H})| = \boldsymbol{H}^{(t)^{-T}}$$

,

[Formula 130]

$$vol(\widehat{\boldsymbol{B}}) = \log|\det(\boldsymbol{H})| \le Tr\left[\boldsymbol{H}^{(t)^{-1}}\boldsymbol{H}\right] + const = Tr\left[\boldsymbol{F}^{(t)}\widehat{\boldsymbol{B}}\widehat{\boldsymbol{B}}^{T}\right] + const,$$

where

[Formula 131]

$$\boldsymbol{F}^{(t)} = \boldsymbol{H}^{(t)^{-1}}$$

and const is a constant term. Thus, by replacing

[Formula 132]

$$vol(\widehat{\boldsymbol{B}})$$

with

[Formula 133]

$$Tr\left[\boldsymbol{F}^{(t)}\widehat{\boldsymbol{B}}\widehat{\boldsymbol{B}}^{T}\right]$$

,

all penalty terms are written collectively as

[Formula 134]

$$\frac{\alpha}{2}vol(\widehat{\boldsymbol{B}}) + \frac{\beta}{2}nonorth(\widehat{\boldsymbol{B}}) \le \frac{\alpha}{2}Tr(\boldsymbol{F}^{(t)}\widehat{\boldsymbol{B}}\widehat{\boldsymbol{B}}^{T}) + \frac{\beta}{2}Tr(\boldsymbol{\Lambda}^{(t)}\widehat{\boldsymbol{B}}\widehat{\boldsymbol{B}}^{T}) + const$$

$$= \frac{1}{2}Tr(\boldsymbol{V}\widehat{\boldsymbol{B}}\widehat{\boldsymbol{B}}^{T}) + const,$$

where

[Formula 135]

$$\boldsymbol{V} = \alpha\boldsymbol{F}^{(t)} + \beta\boldsymbol{\Lambda}^{(t)}.$$

Accordingly,

[Formula 136]

$$\widehat{B}^{(t+1)}$$

can be updated by solving the following:

[Formula 137]

$$\widehat{B}^{(t+1)} = \arg\min_{\widehat{B}} \frac{1}{2} Tr\left[W\left(\widehat{A} - C^{(t)}\widehat{B}\right)\left(\widehat{A} - C^{(t)}\widehat{B}\right)^T\right] + \frac{1}{2} Tr\left(V\widehat{B}\widehat{B}^T\right) \qquad (S14)$$

$$s.t.\,\widehat{B} = BL, B \geq 0.$$

In order to organize complicated constraint conditions, a constraint

[Formula 138]

$$\widehat{B} = BL$$

is incorporated into an objective function using Lagrange's undetermined multiplier as follows:

[Formula 139]

$$Z \in \mathbb{R}^{K \times M}$$

, thereby solving this problem in a framework of ADMM.

[Formula 140]

$$\left(B^{(t+1)}, \widehat{B}^{(t+1)}\right) = \arg\min_{B,\widehat{B}} \max_{Z} \left\{ f(\widehat{B}) + Tr\left[Z^T\left(\widehat{B} - BL\right)\right] - \frac{1}{2\mu}\|Z - Z'\|_F^2 \right\},$$

$$s.t.\,B \geq 0,$$

where

[Formula 141]

$$f(\widehat{B}) \equiv \frac{1}{2} Tr\left[W\left(\widehat{A} - C^{(t)}\widehat{B}\right)\left(\widehat{A} - C^{(t)}\widehat{B}\right)^T\right] + \frac{1}{2} Tr\left(V\widehat{B}\widehat{B}^T\right)$$

and $\mu$ is a hyper parameter for ADMM (here, $\mu$ = 1 was consistently used). Furthermore, Z' represents Z at previous timing. When

[Formula 142]

$$Z = Z' + \mu\left(\widehat{B} - BL\right), \qquad (S15)$$

, the objective function provides the following maximized with respect to Z:

[Formula 143]

$$g\left(\boldsymbol{B}, \widehat{\boldsymbol{B}}; \boldsymbol{Z}\right) \equiv f\left(\widehat{\boldsymbol{B}}\right) + \frac{\mu}{2} \left\| \widehat{\boldsymbol{B}} - \boldsymbol{BL} + \frac{1}{\mu} \boldsymbol{Z} \right\|_F^2, \boldsymbol{B} \geq 0,$$

This may be optimized with respect to

[Formula 144]

$$\left(\boldsymbol{B}, \widehat{\boldsymbol{B}}\right)$$
.

[Formula 145]

$$\left(\boldsymbol{B}, \widehat{\boldsymbol{B}}, \boldsymbol{Z}\right)$$

was optimized cyclically and an algorithm was summarized as Algorithm 3.

[Formula 146]

**Algorithm3:** ADMM for solving the optimization of Eq. S14

**input:** $\boldsymbol{B}^{(t)}, \widehat{\boldsymbol{B}}^{(t)}$, hyperparameter $\mu$, function $g(\boldsymbol{B}, \widehat{\boldsymbol{B}})$

**output:** $\boldsymbol{B}^{(t+1)}, \widehat{\boldsymbol{B}}^{(t+1)}$

**initialize:** $q = 0, \boldsymbol{B}_q \leftarrow \boldsymbol{B}^{(t)}, \widehat{\boldsymbol{B}}_q \leftarrow \widehat{\boldsymbol{B}}^{(t)}, \boldsymbol{Z}_q \leftarrow \boldsymbol{0}$

**repeat until convergence:**

$$\widehat{\boldsymbol{B}}_{q+1} = \underset{\widehat{\boldsymbol{B}}}{\operatorname{argmin}} \, g(\widehat{\boldsymbol{B}}; \boldsymbol{B}_q, \boldsymbol{Z}_q), \text{ solved by Eq. S16}$$

$$\boldsymbol{B}_{q+1} = \underset{\boldsymbol{B} \geq 0}{\operatorname{argmin}} \, g(\boldsymbol{B}; \widehat{\boldsymbol{B}}_{q+1}, \boldsymbol{Z}_q), \text{ solved by Eq. S17}$$

$$\boldsymbol{Z}_{q+1} = \boldsymbol{Z}_q + \mu(\widehat{\boldsymbol{B}}_{q+1} - \boldsymbol{B}_{q+1}\boldsymbol{L})$$

$$q \leftarrow q + 1$$

**if** $\left\| \widehat{\boldsymbol{B}}_q - \boldsymbol{B}_q\boldsymbol{L} \right\|_F^2 < 10^{-6}$:

**end**

$$\boldsymbol{B}^{(t+1)} \leftarrow \boldsymbol{B}_q, \widehat{\boldsymbol{B}}^{(t+1)} \leftarrow \widehat{\boldsymbol{B}}_q.$$

As objective function:

[Formula 147]

$$g(\widehat{\boldsymbol{B}}; \boldsymbol{B}_q, \boldsymbol{Z}_q)$$

is optimization of a quadratic function without restraint condition about

[Formula 148]

$$\widehat{\boldsymbol{B}}$$
,

it is minimized by the following:

[Formula 149]

$$\frac{\partial g}{\partial \widehat{\boldsymbol{B}}} = \left( \boldsymbol{C}^{(t)^T} \boldsymbol{W} \boldsymbol{C}^{(t+1)} + \boldsymbol{V} + \mu \boldsymbol{I}_K \right) \widehat{\boldsymbol{B}} - \boldsymbol{C}^{(t)^T} \boldsymbol{W} \widehat{\boldsymbol{A}} - \boldsymbol{B}_q \boldsymbol{L} + \frac{1}{\mu} \boldsymbol{Z} \equiv \boldsymbol{0}$$

$$\widehat{\boldsymbol{B}}_{q+1} = \left( \boldsymbol{C}^{(t)^T} \boldsymbol{W} \boldsymbol{C}^{(t)} + \boldsymbol{V} + \mu \boldsymbol{I}_K \right)^{-1} \left( \boldsymbol{C}^{(t)^T} \boldsymbol{W} \widehat{\boldsymbol{A}} + \boldsymbol{B}_q \boldsymbol{L} - \frac{1}{\mu} \boldsymbol{Z} \right), \qquad (S16)$$

Furthermore,

[Formula 150]

$$g\left( \boldsymbol{B}; \widehat{\boldsymbol{B}}_{q+1}, \boldsymbol{Z}_q \right)$$

can be solved by NNLS and is updated as
[Formula 151]

$$\boldsymbol{B}_{q+1}{}^T = NNLS\left( \left( \widehat{\boldsymbol{B}}_{q+1} + \frac{1}{\mu} \boldsymbol{z} \right)^T, \boldsymbol{L}^T \right), \qquad (S17)$$

As a result of the foregoing, the following update formula for solving the original problem Eq. S11 was obtained:

[Formula 152]

$$\left( \widehat{\boldsymbol{B}}, \boldsymbol{B}, \boldsymbol{C} \right)$$

Finally, update of $V = \alpha F^{(t)} + \beta \Lambda^{(t)}$ is considered. Regarding $F^{(t)}$, it can be determined easily as
[Formula 153]

$$\boldsymbol{F}^{(t)} = \left( \widehat{\boldsymbol{B}}^{(t)} \widehat{\boldsymbol{B}}^{(t)^T} + \tau \boldsymbol{I}_K \right)^{-1}, \qquad (S18)$$

Regarding $\Lambda^{(t)}$, by combining strict orthogonal conditions, which are specifically

[Formula 154]

$$\widehat{\boldsymbol{B}} \widehat{\boldsymbol{B}}^T = diag\left( \widehat{\boldsymbol{B}} \widehat{\boldsymbol{B}}^T \right) \equiv \boldsymbol{D}, \ \beta = 1$$

and
[Formula 155]

$$\frac{\partial f\left(\widehat{\boldsymbol{B}};\boldsymbol{C}^{(t)}\right)}{\partial \widehat{\boldsymbol{B}}} = \boldsymbol{C}^{(t)^T}\boldsymbol{W}\left(\boldsymbol{C}^{(t)}\widehat{\boldsymbol{B}} - \widehat{\boldsymbol{A}}\right) + \left(\alpha \boldsymbol{F}^{(t)} + \boldsymbol{\Lambda}^{(t)}\right)\widehat{\boldsymbol{B}} \equiv 0. \qquad (S19)$$

, and by multiplying the following from the right side of Eq. S19:

[Formula 156]

$$\widehat{\boldsymbol{B}}^T$$

,

, $\Lambda^{(t)}$ can be obtained as
[Formula 157]

$$\boldsymbol{C}^{(t)^T}\boldsymbol{W}\left(\boldsymbol{C}^{(t)}\boldsymbol{D} - \widehat{\boldsymbol{A}}\widehat{\boldsymbol{B}}^T\right) + \left(\alpha \boldsymbol{F}^{(t)} + \boldsymbol{\Lambda}^{(t)}\right)\boldsymbol{D} \equiv 0,$$
$$\boldsymbol{\Lambda}^{(t)} = \boldsymbol{C}^{(t)^T}\boldsymbol{W}\left(\widehat{\boldsymbol{A}}\widehat{\boldsymbol{B}}^T\boldsymbol{D}^{-1} - \boldsymbol{C}^{(t)}\right) - \alpha \boldsymbol{F}^{(t)}. \qquad (S20)$$

Using Eq. S18 and Eq. S20,
[Formula 158]

$$\boldsymbol{V} = \alpha(1 - \beta)\left(\widehat{\boldsymbol{B}}^{(t)}\widehat{\boldsymbol{B}}^{(t)^T} + \tau \boldsymbol{I}_K\right)^{-1} + \beta \boldsymbol{C}^{(t)^T}\boldsymbol{W}\left(\widehat{\boldsymbol{A}}\widehat{\boldsymbol{B}}^T\boldsymbol{D}^{-1} - \boldsymbol{C}^{(t)}\right). \qquad (S21)$$

is given. Using the foregoing, an algorithm for solving the problem Eq. S11 is obtained as follows:
[Formula 159]

**Algorithm 4**: Pseudo-code for the second NMF (solving optimization Eq. S11)

**Input**: output of the first NMF (sample-wise FA; $\widetilde{A} \in \mathbb{R}_+^{N \times M}$ , basis-fragment spectra; $S \in \mathbb{R}_+^{M \times D}$), basis-polymer number; $K$, weights for penalty terms; $(\alpha, \beta)$, weight for outliers; $p$

**Output**: polymer weight-fraction; $C \in \mathbb{R}_+^{N \times K}$, FAs of unit-weight basis-polymers $B \in \mathbb{R}_+^{K \times M}$

**Initialization**

calculate $L$ via Cholesky decomposition of $SS^T$

set $\widehat{A} = \widetilde{A}L$

initialize $\widehat{B}^{(0)}$ by selecting $K$-rows of $\widehat{A}$ via VCA algorithm

set $B^{(0)} = \widehat{B}^{(0)}L^{-1}$

initialize $C^{(0)}$ by $C^{(0)^T} = FCLS(\widehat{A}^T, \widehat{B}^{(0)^T})$

initialize $W$ by Eq. S12

initialize $V$ based on $B^{(0)}$ and $C^{(0)}$ by Eq. S21

**Repeat until criteria for convergence satisfied:**

update $\widehat{B}, B$ by algorithm 3

update $C$ by Eq. S13

update $W, V$ by Eq. S12 and S21, respectively

(Projection of Test Data onto Hyperplane Spanned by S and B)

After S and B are inferred from the data set, the following data not having been used for the inference of S and B (here, called test data) is projected onto a hyperplane spanned by S and B by a method described herein:

[Formula 160]

$$X_{test} \in \mathbb{R}_+^{T \times D}$$

First, by the projection onto an S-hyperplane,

[Formula 161]

$$A_{test} \in \mathbb{R}_+^{T \times M}$$

is obtained. Specifically, a total sum is determined with respect to temperature zones

[Formula 162]

$$A_{test}{}^T = NNLS\left(R^{-\frac{1}{2}}X_{test}{}^T, R^{-\frac{1}{2}}S^T\right),$$

which is converted to

[Formula 163]

$$\widetilde{A}_{test} \in \mathbb{R}_+{}^{1 \times M}$$

Then, by performing projection onto a B-hyperplane and normalization,

[Formula 164]

$$C_{test} \in \mathbb{R}_+{}^{1 \times K}$$

is obtained. Specifically,

[Formula 165]

$$C_{test}{}^T = NNLS\left(L^T\widetilde{A}_{test}{}^T, L^TB^T\right),$$

$$C_{test} \leftarrow \frac{C_{test}}{\|C_{test}\|_1}.$$

Cited literatures are as follows:

[Table 1]

1. S. Boyd, N. Parikh, E. Chu, B. Peleato, J. Eckstein, Distributed optimization and statistical learning via the alternating direction method of multipliers. Found. Trends Mach. Learn. 3, 1-122 (2010).
2. X. Fu, W. Ma, K. Huang, N. D. Sidiropoulos, ROBUST VOLUME MINIMIZATION-BASED MATRIX FACTORIZATION VIA ALTERNATING OPTIMIZATION Department of ECE , University of Minnesota , Minneapolis , MN , USA Department of EE , The Chinese University of Hong Kong , Shatin , N . T ., Hong Kong. Icassp 2016. MI, 2534-2538 (2016).
3. X. Fu, K. Huang, B. Yang, W. K. Ma, N. D. Sidiropoulos, Robust Volume Minimization-Based Matrix Factorization for Remote Sensing and Document Clustering. IEEE Trans. Signal Process. 64, 6254-6268 (2016).

(continued)

4. J. M. B. Dias, J. M. P. Nascimento, Vertex component analysis: A geometric-based approach to unmix hyperspectral data. Signal Image Process. Remote Sens. 43, 415-439 (2006).

Next, model polymer spectrum (hereinafter may be referred to as "M spectrum") calculation in step S5 will be described in detail. In the step S5, the spectra of the model polymers formed from only one type of polyad (M spectra) are reconstructed from calculation result described above. The method is to obtain the matrix product of a matrix representing a basis spectrum obtained by the first NMF process, and a matrix representing a feature vector of a model polymer obtained by the second NMF process.

The model polymer spectra (M spectra) are calculated by multiplying the basis spectrum by a matrix (like a coefficient) representing the feature vector characteristic of each model polymer. Specifically, the M spectra are calculated as the matrix product of a matrix representing the basis spectrum and a matrix representing the feature vector of the model polymer.

Specifically, when X = AS in the first NMF process, this is factorized into X = AS = (CB)S in the second NMF process. Furthermore, by being interpreted as C(BS), this can be factorized into the mass ratio C of a model compound and the spectrum BS of the model polymer. The BS corresponds to the M spectrum.

It can be said that the M spectra calculated by the present step infer the mass spectrum of a sample containing only the model polymer. Step S6 performs identification to determine which polyad each M spectrum can be attributed to.

As have already been described, the number of polyads is determined based on the number of combinations which can be theoretically made, and every M spectrum can be attributed to each polyad in principle. However, while the details are described below, one or more M spectra cannot be attributed to any polyad.

In the step S6, it is judged whether or not an M spectrum not attributed to any polyad is present. When an M spectrum which cannot be attributed to any polyad is present (step S6: YES), the number K of variants of polyads is changed, and the first NMF process and the second NMF process are repeated again.

When an M spectrum which cannot be attributed to any polyad is present, it is considered that at least one or more variants of polyads are not actually present, or enough polyads are not contained in the reference samples as one of the reasons.

As the former case, about polymer triads obtained from monomers A, B and C, for example, a case where A and B do not have properties of alternating copolymerization will be considered. On the assumption that "AB{AB}" is present as one variant of the triads, when the number K of variants is determined and M spectra are obtained, an M spectrum derived from (attributed to) "AB{AB}" cannot be obtained. The reason for this is that a polymer having such triad is not contained in a sample.

Therefore, as a result, one or more M spectra are not attributed to any polyad.

In such case, 1 is subtracted from K, and then the first NMF process and the second NMF process are performed again, thereby being able to make modifications.

Specifically, when an M spectrum which cannot be attributed to any polyad is present, 1 is mechanically subtracted from K, and M spectra are obtained again. Thus, even if a fact that A and B do not have properties of alternating copolymerization is unknown, more accurate analysis result is obtained.

As described above, when the present sequence analysis method has steps S5 and S6, polyad variants which cannot be present by e.g. individual combinations of monomers are incorporated into K. Even when the reason is unknown, the validity of analysis can be evaluated and modifications can be made only by confirming whether or not an M spectrum can be attributed to the determined polyad.

Even when "AB" polyad, for example, is actually present, when the polyad is not sufficiently contained in a sample (typically, reference sample), the state may be the same as the above. In this case, the samples are added, and modifications can be made by acquiring a data matrix and performing the first NMF process and the second NMF process again. Even in this case, the first NMF process and the second NMF process may be performed after 1 is subtracted from K without changing the reference samples.

On the other hand, when every M spectrum is attributed to each of polyads (step S6: NO), the next step (S7) is performed.

While the details are described below, polyad content ratios are inferred by projecting a feature vector of the sample into the K-1 dimensional simplex in the present sequence analysis method, and the "end member" as a vertex of the K-1 dimensional simplex is required. This "end member" is defined by a feature vector of a polymer (model polymer) formed only from a polyad. However, it is frequently difficult to actually prepare a model polymer as the reference sample.

In triad analysis when the sample set includes three types of monomers, A, B and C, for example, the number K of variants is 13 in principle and feature vectors of 13 types of "model polymers" including three types of homopolymers are required as the "end members." In many cases, however, it is not realistic to synthesize these 13 types of model (co) polymers by precisely controlling sequences of units and acquiring mass spectra.

In the present sequence analysis method, contrarily, the first NMF process and the second NMF process are combined as described above to estimate feature vectors of the "end members" and one feature thereof is not to require to actually measure mass spectra of the model polymers.

Actual examples of M spectrum analysis are described with test results in steps S5 and 6 so far.

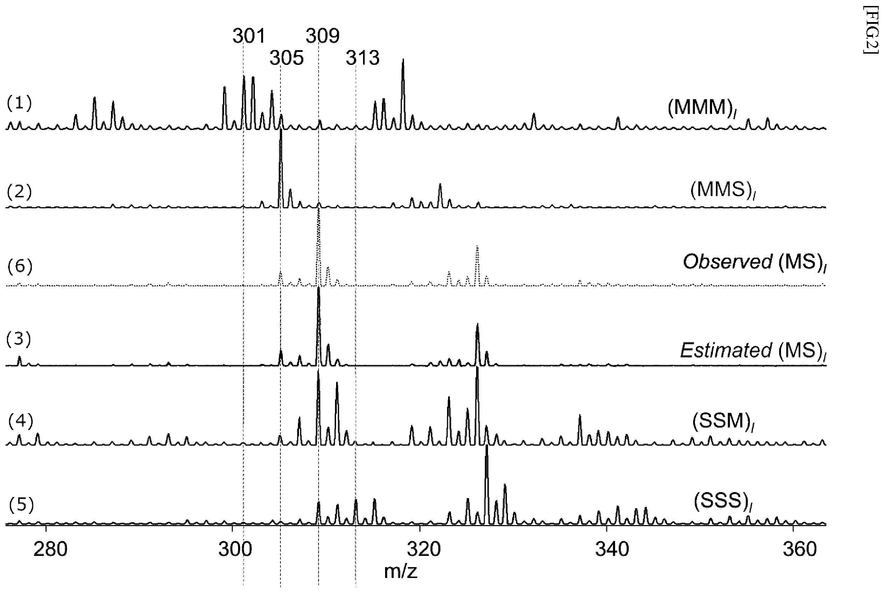

Fig. 2 shows M spectra calculated from respective matrices obtained by acquiring data matrices from samples synthesized using methyl methacrylate (M) and styrene (S) as monomers and performing the first NMF process and the second NMF process. Experimental procedure will now be described.

First, samples were synthesized by adding methyl methacrylate, styrene and a polymerization initiator (dimethyl 2,2'-azobis(isobutyrate)) to a reaction container, and heating the obtained mixture for a predetermined time. The amounts of methyl methacrylate and styrene added, heating temperature and reaction time were adjusted to various values to produce polymers with different composition, and the polymers were used as the samples. Detail procedure, etc. are the same as in Examples described below, and the description is omitted here. Polymerization conditions are as shown in Tables below.

[Table 2]

| Table1 | M initial fraction | S initial fraction | Time (h) | Temp(°C) |
|---|---|---|---|---|
| sample1 | 0.05 | 0.95 | 1 | 60 |
| sample2 | 0.1 | 0.9 | 1 | 40 |
| sample3 | 0.1 | 0.9 | 1 | 60 |
| sample4 | 0.15 | 0.85 | 1 | 60 |
| sample5 | 0.15 | 0.85 | 1 | 60 |
| sample6 | 0.2 | 0.8 | 1 | 60 |
| sample7 | 0.2 | 0.8 | 1 | 60 |
| sample8 | 0.3 | 0.7 | 1 | 40 |
| sample9 | 0.3 | 0.7 | 1 | 60 |
| sample10 | 0.35 | 0.65 | 1 | 40 |
| sample11 | 0.35 | 0.65 | 1 | 60 |
| sample12 | 0.4 | 0.6 | 1 | 40 |
| sample13 | 0.4 | 0.6 | 1 | 60 |
| sample14 | 0.4 | 0.6 | 1 | 60 |
| sample15 | 0.45 | 0.55 | 1 | 60 |
| sample16 | 0.5 | 0.5 | 1 | 40 |
| sample17 | 0.5 | 0.5 | 1 | 60 |
| sample18 | 0.55 | 0.45 | 1 | 60 |
| sample19 | 0.6 | 0.4 | 1 | 40 |
| sample20 | 0.6 | 0.4 | 1 | 60 |
| sample21 | 0.65 | 0.35 | 1 | 60 |
| sample22 | 0.7 | 0.3 | 1 | 40 |
| sample23 | 0.7 | 0.3 | 1 | 60 |
| sample24 | 0.8 | 0.2 | 1 | 60 |
| sample25 | 0.8 | 0.2 | 1 | 60 |
| sample26 | 0.85 | 0.15 | 1 | 60 |
| sample27 | 0.9 | 0.1 | 1 | 60 |
| sample28 | 0.9 | 0.1 | 1 | 60 |
| sample29 | 0.95 | 0.05 | 1 | 60 |

(continued)

| Table1 | M initial fraction | S initial fraction | Time (h) | Temp(°C) |
|---|---|---|---|---|
| sample30 | 1 | 0 | 1 | 60 |
| sample31 | 0 | 1 | 1 | 60 |

The "M initial fraction" and "S initial fraction" in the table represent the added amount ratios of methyl methacrylate and styrene, respectively, and "Time (h)" and "Temp (C°)" represent polymerization time and heating temperature, respectively. Thus, 31 types of samples different from each other were produced.

As described above, the number of samples was 31, the temperature range of mass spectrometry was 200-450°C, and the m/z range was 50-410. Other hyper parameters are as follows.

[Table 3]

| First NMF | | | | | Second NMF | | |
|---|---|---|---|---|---|---|---|
| $w_o$ | Merging threshold | Initial $M$ | Prior | iteration | $K$ | $\alpha = \beta$ | $p$ |
| 0.2 | 0.99 | 30 | Exp | 3000 | 5 | 0.05 | 1 |

In this experiment, the number of types of monomers contained in the monomer set is two types, methyl methacrylate and styrene. The length of polyads was 3 (triad analysis), and thus the variants of polyads were all 5 types, MMM, MMS, MS{MS}, SSM and SSS assumed as theoretical combinations (the number K of variants = 5).

Fig. 2 is calculated M spectra. The spectra (1) to (5) in Fig. 2 are respective calculated M spectra. Here, a label to show which polyad an M spectrum is derived from is not given in the first NMF process and the second NMF process. Even if a label is not given, the subsequent analysis is not affected. On the other hand, by attributing an M spectrum to a polyad, the validity of analysis conditions, etc. can be checked, and consequently more accurate analysis can be made.

As have already been described, the step S6 is a step of identifying a polyad to which an M spectrum is attributed.

The M spectrum is attributed to any of polyads in principle, which is identified in step S6. While the identification method is not particularly restricted, identification is preferably made by comparing the sum of mass numbers of units forming a polyad and m/z at peaks of an M spectrum as an aspect.

To describe the case in Fig. 2, the sum of mass numbers of units forming a polyad is 300 in MMM, 304 in MMS, 304 and 308 in MS (MS), 308 in SSM and 312 in SSS. In contrast, the peaks in the M spectra are 300 + 1 in the spectrum (1), 304 + 1 in (2), 304 + 1 and 308 + 1 in (3), 308 + 1 in (4) and 312 + 1 in (5), and correspond to protonated peaks in the above polyads.

According to the foregoing, the M spectra in the order of (1) to (5) are identified to be MMM, MMS, MS (MS), SSM and SSS, respectively. The labels of the respective spectra in Fig. 2 were given as a result in the present step.

The spectrum (6) in Fig. 2 represents a mass spectrum obtained from an M/S alternating copolymer which was actually polymerized. It is found that the spectrum almost agree with the calculated spectrum (3).

The M spectra obtained as a result of the first NMF process and the second NMF process in the present sequence analysis method are almost the same as the mass spectra which are actually measured as described above, and thus identification can be easily made based on the structures and mass numbers of polyads in the present step.

Referring back to Fig. 1, then, the feature vector of the model polymer is defined as an end member, and the K-1 dimensional simplex including all of the feature vectors (each corresponding to one sample) is determined as step S7. In the present sequence analysis method, the K-1 dimensional simplex is determined by inferred end members by the second NMF process regardless of whether or not the end members are included in reference samples.

When the number K of variants is equal to or greater than 3, it is preferred that at least one of the feature vectors of the reference samples be present in each region external to a hypersphere inscribed in the K-1 dimensional simplex or the reference samples contain at least one of the end members. According to the foregoing, more accurate analysis result is obtained.

Here, each position in the region of the K-1 dimensional simplex represents an end member content ratio in terms of mass. Therefore, the region external to a hypersphere inscribed in the K-1 dimensional simplex schematically represents a region where any end member, specifically polyad content is equal to or greater than a predetermined amount. When the feature vector of a reference sample is present at such position, this specifically means that the sample set contains a reference sample in which any polyad content is equal to or greater than a predetermined amount. As one of the aspects, a reference sample contains an end member.

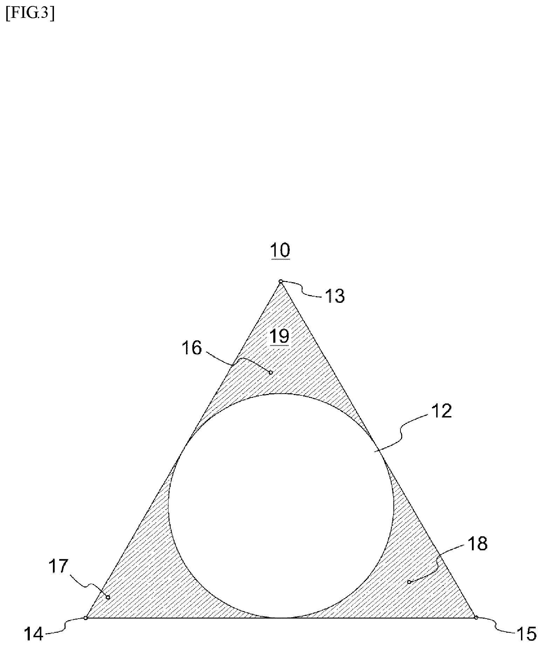

Fig. 3 is a conceptual view showing a K-1 dimensional simplex (two-dimensional simplex, a triangle) when K is 3. The K-1 dimensional simplex 10 is a triangle with vertexes defined by end members 13, 14, and 15 determined by reference samples 16, 17, and 18 respectively.

As shown in Fig. 3, if all the reference samples 16, 17, and 18 are present in a region 19 (hatched) external to a hypersphere 12 (in this case, a "circle") inscribed in the K-1 dimensional simplex, high-precision quantitative analysis result is obtained.

Next, a distance between each of K end members and a feature vector of an inference target sample is calculated to infer polyad content ratios of the inference target sample (step S8).

The above distance is defined by the Riemannian metric distance in consideration of non-orthogonality of the basis spectrum obtained by the first NMF process.

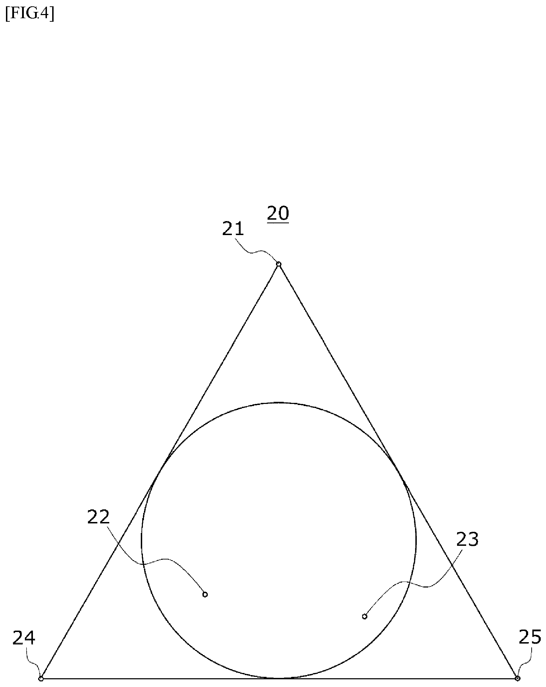

When the reference sample contains at least one end member (when at least one reference sample is an end member), a feature vector of another reference sample may be present in a region inside a hypersphere inscribed in the K-1 dimensional simplex. Like Fig. 3, Fig. 4 is a conceptual view showing a K-1 dimensional simplex (two-dimensional simplex, a triangle) when K is 3. The K-1 dimensional simplex 20 is a triangle with vertexes defined by a reference sample 21 as an end member, and by end members 24 and 25 determined by different reference samples 22 and 23 respectively. A difference from Fig. 3 is that the reference samples 22 and 23 are present in a region inside a hypersphere inscribed in the K-1 dimensional simplex.

Even if at least one reference sample is an end member, a different reference sample may still be present on a hypersphere inscribed in the K-1 dimensional simplex or in a region external to the hypersphere. Specifically, in this case, the different reference sample may be present at any position.

In terms of achieving more excellent effect of the present invention, it is preferable that the different reference sample contain 20% by mass or more, more preferably, 40% by mass or more of a component of an end member different from the reference sample as an end member (a component of a different end member).

According to the present sequence analysis method, even for a polymer which is not easy to prepare a reference sample, accurate sequence analysis can be made without particular preparatory process by a simple procedure. The present sequence analysis method achieves significant reduction in time required for obtaining result, particularly when being applied to quality control of a polymer synthesized from a plurality of monomers, investigation of a cause for failure, etc.

The polymer to which the present sequence analysis method can be applied is not particularly restricted, and both synthetic polymers and natural polymers may be used. While a (co)polymer synthesized based on monomers having an ethylenically unsaturated bond is described in Examples described below, the (main chain) structure of the polymer is not limited thereto. The polymerization method is not particularly restricted, and a polymer synthesized by any of methods such as addition polymerization, ring-opening polymerization, polycondensation, polyaddition and addition polymerization may be used. The reference sample and the inference target sample may have different synthesis method (polymerization method) as long as the samples are synthesized from the same monomer set.

For example, the reference sample may be a sample prepared by common radical polymerization while changing polymerization conditions to various conditions, and the inference target sample may be a sample polymerized while making precise control by another method (for example, living polymerization such as atom transfer radical polymerization and reversible addition-fragmentation chain transfer (RAFT) polymerization), etc.

One application example of the present sequence analysis method includes application to sequence analysis for (photo)resist resin. It is known that there is correlation between the developability of a resist resin and a polyad sequence, and the development of a resist resin having more excellent developability, investigation of a cause for development failure of a resist resin, etc. become easier by performing sequence analysis for the resist resin using the present sequence analysis method.

The resist resin to which the present sequence analysis method can be applied is not particularly limited. Examples of resist resins include resins synthesized from the following monomers.

3-Hydroxy-1-adamantyl methacrylate, 1-adamantyl acrylate, 1-adamantyl methacrylate, 2-methyl-2-adamantyl methacrylate, 2-methyladamantan-2-yl acrylate, 2-ethyl-2-adamantyl methacrylate, 2-ethyl-2-adamantane acrylate, dicyclopentanyl acrylate, 2-isopropyladamantan-2-yl acrylate, tetrahydrodicyclopentadienyl methacrylate, 5-methacroyloxy-2,6-norbornane carbolactone, β-hydroxy-γ-butyrolactone methacrylate, 1-ethylcyclopentyl methacrylate, α-methacryloxy-γ-butyrolactone, 1-ethylcyclohexyl methacrylate, 1-methylcyclopentyl methacrylate, 4-acetoxystyrene, 2-oxo-2-(2,2,3,3,3-pentafluoropropoxy)ethyl methacrylate, 3-hydroxy-1-adamantyl acrylate, (adamantan-1-yloxy)methyl methacrylate, 2-isopropyl-2-adamantyl methacrylate, 1,3-adamantanediol diacrylate, 1,3-adamantanediol dimethacrylate, 1-methyl-1-ethyl-1-adamantylmethanol methacrylate, 1,1-diethyl-1-adamantylmethanol methacrylate, 5,7-dimethyl-1,3-adamantanediol diacrylate, 5,7-dimethyl-1,3-adamantanediol dimethacrylate, 5-ethyl-1,3-diadamantanediol diacrylate, 5-ethyl-1,3-diadamantanediol dimethacrylate, 2-methyl-2-propenoic acid 2-oxo-2-[(5-oxo-4-oxatricyclo [4.3.1.1³,⁸]undec-2-yl)oxy]ethyl ester, 2-propenoic acid, 2-methyl-,2-[(hexahydro-2-oxo-3,5-methano-2H-cyclopenta [b]furan-6-yl)oxy]-2-oxoethyl ester, 2-(2,2-difluoroethenyl)bicyclo[2.2.1]heptane, 6-methacryloyl-6-azabicyclo[3.2.0] heptan-7-one, 2-propenoic acid, (3R, 3aS, 6R, 7R, 8aS)-octahydro-3,6,8,8-tetramethyl-1H-3a,7-methanoazulen-6-ol ester, 2-propenoic acid, (3R, 3aS, 6R, 7R, 8aS)-octahydro-3,6,8,8-tetramethyl-1H-3a,7-methanoazulen-6-yl ester, 2-cyclohexylpropan-2-yl methacrylate, 1-isopropylcyclohexyl methacrylate, 1-methylcyclohexyl methacrylate, 1-ethylcyclopentyl acrylate, 1-methylcyclohexyl acrylate, tetrahydropyranyl methacrylate, tetrahydro-2-furanyl methacrylate, (3-methyl-5-oxooxolan-3-yl)2-methyl prop-2-enoate, 2-oxotetrahydrofuran-3-yl acrylate, (5-oxotetrahydrofuran-2-yl)methyl methacrylate, (2-oxo-1,3-dioxolan-4-yl)methyl methacrylate, 1-ethoxyethyl methacrylate, N-(methoxymethyl)methacrylamide, N-isopropylmethacrylamide, 2-(bromomethyl)acrylic acid ethyl, 2-(bromomethyl) acrylic acid methyl, N-butyl-2-(bromobutyl) acrylate, 2,2,3,3,4,4,4-heptafluorobutyl methacrylate, 2,5-dimethylhexan-2,5-diyl bis(2-methyl methacrylate), 2-(trifluoromethanesulfonamide)ethyl methacrylate, 3-[dimethoxy(methyl)silyl]propyl acrylate, 2,3-dihydroxypropyl acrylate, 9H-fluorene-9,9-dimethanol dimethacrylate, 9,9-bis[(acryloyloxy)methyl]fluorene, 9-anthrylmethyl methacrylate, 4-hydroxyphenyl methacrylate, 4-(4-acryloyloxybutoxy)benzoic acid, 3-(4-hydroxy-phenoxy)propyl acrylate, 10-([1,1'-biphenyl]-2-yloxy)decyl acrylate, 2-vinylnaphthalene, 4-tert-butoxystyrene, 4-isopropenyl phenol, 4-(1-ethoxyethoxy)styrene, 3,4-diacetoxystyrene, 4-allyloxystyrene, 3-tert-butoxystyrene, 2-acetoxystyrene, 4-ethenyl-1,2-bis(1-ethoxyethoxy)benzene, tetrahydro-2-[4-(1-methylethenyl)phenoxy]furan, 2-[(4-vinylphenoxy)methyl]oxirane, 3,5-diacetoxystyrene, 2,3-difluoro-4-vinylphenol, 3-fluoro-4-vinylphenol, 1,1,2,2-tetramethylpropyl acrylate, 1-ethenyl-4-propan-2-yloxybenzene, 4-vinylphenyl benzoate, 1-ethylhexyl methacrylate, 2-isopropyl-2-adamantyl methacrylate, 3-hydroxy-1-adamantyl methacrylate, 1,1-dimethylpentyl methacrylate, 1,1-dimethylhexyl methacrylate, neopentyl methacrylate and 2,2,2-trifluoroethyl methacrylate.

Specific examples of combinations of monomers include γ-butyrolactone (meth)acrylate/2-methyl-2-adamantyl (meth)acrylate/3-hydroxy-1-adamantyl (meth)acrylate and 4-hydroxystyrene/2-methyl-2-adamantyl (meth)acrylate/styrene and the like.

[Sequence analyzing device (first embodiment)]

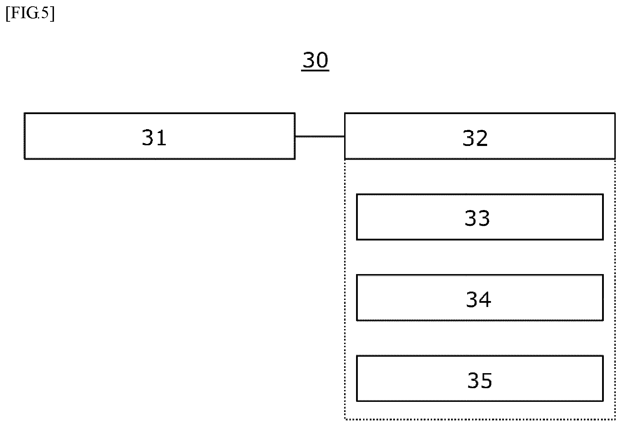

A sequence analyzing device according to an embodiment of the present invention will now be described with reference to drawings. Fig. 5 is a hardware configuration view of the sequence analyzing device according to one embodiment of the present invention.

The sequence analyzing device 30 includes a mass spectrometer 31 and an information processing device 32. The information processing device 32 has a processor 33, a storage device 34, and an input-output interface (I/F) 35 to connect a display device and an input device, not shown. The mass spectrometer 31 and the information processing device 32 are configured in such a manner that data can be transferred therebetween.

Examples of the processor 33 include a microprocessor, a processor core, a multiprocessor, an ASIC (application-specific integrated circuit), an FPGA (field programmable gate array), and GPGPUs (general-purpose computing on graphics processing units), and the like.

The storage device 34 has the function of storing various types of programs and data in a transitory manner and/or a non-transitory manner, and provides a work area for the processor 33.

Examples of the storage device 34 include a ROM (read only memory), a RAM (random access memory), an HDD (hard disk drive), a flash memory, an SSD (solid state drive) and the like.

The input device connected to the input-output I/F 35 can accept various types of information input and also can accept input of a command to the sequence analyzing device 30. Examples of the input device may be a keyboard, a mouse, a scanner, and a touch panel, and the like.

The display device connected to the input-output I/F 35 can display a status of the sequence analyzing device 30, progress of analysis, and sequence analysis result, and the like. Examples of the display device may include a liquid crystal display and an organic EL (electro luminescence) display, and the like.

The display device may be configured as a device integrated with the input device. In this case, the display device may be configured as a touch panel display and to provide a GUI (graphical user interface).

The information processing device 32, which includes the processor 33, the storage device 34 and the input-output I/F 35 allowing communication of data therebetween through a data bus, is typically a computer.

The mass spectrometer 31 is typically a mass spectrometer including a "DART" ion source, a sample heater, and a time-of-flight type mass spectrometer instrument. Both the ion source and the mass spectrometer instrument of the mass spectrometer 31 are non-restrictive examples and the configuration of the mass spectrometer provided to the sequence analyzing device is not limited to that described above.

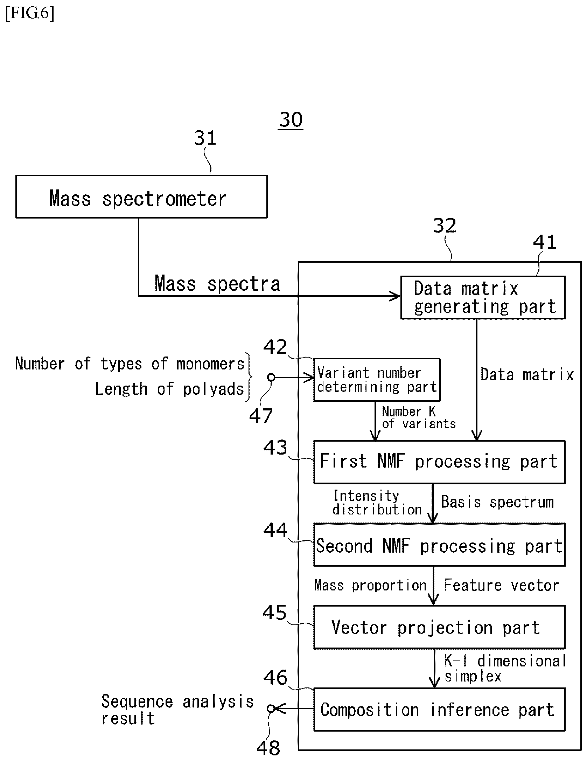

Fig. 6 is a functional block diagram of the sequence analyzing device 30. The sequence analyzing device 30 includes a mass spectrometer 31 that performs mass spectrometry on samples, and the information processing device 32 that processes a mass spectrum acquired by the mass spectrometer 31.

The processor 33 executes the program stored in the storage device 34 of the information processing device 32 to control the mass spectrometer 31.

The mass spectrometer 31 ionizes gas components generated as a result of thermal desorption and/or pyrolysis while heating a loaded sample, performs mass spectrometry sequentially, and outputs mass spectra.

The mass spectra acquired in the mass spectrometer 31 are transferred to a data matrix generating part 41 of the information processing device 32. The data matrix generating part 41 is a function realized by causing the processor 33 to execute the program stored in the storage device 34. The data matrix generating part 41 generates a data matrix from two-dimensional mass spectra including the mass spectrum in each row, and transfers the data matrix to a first NMF processing part 43 described later. The details of the data matrix are as have already been described.

The variant number determining part 42 is a function realized by causing the processor 33 to execute the program stored in the storage device 34. The variant number determining part 42 determines the number K of variants depending on the number of types of monomers and the length of polyads acquired from the outside 47 through the input-output I/F 35, and transfers the number to the first NMF processing part 43.

The maximum value of the number K of variants previously determined depending on the number of types of monomers and the length of polyads is stored in the storage device 34, and the variant number determining part 42 refers to the value to determine the number K of variants.

In the present embodiment, while the number of types of monomers and the length of polyads are acquired from the outside 47 through the input-output I/F 35, the number of types of monomers and the length of polyads may be acquired from an external network using communication in addition to the input-output I/F 35.

The maximum value of the number K of variants may be stored as a table of the number of types of monomers and the length of polyads, or may be stored as a function of the number of types of monomers and the length of polyads in the storage device 34.

The first NMF processing part 43 is a function realized by causing the processor 33 to execute the program stored in the storage device 34. The first NMF processing part 43 performs non-negative matrix factorization (NMF) on the data matrix based on the data matrix provided from the data matrix generating part 41 and the number K of variants provided from the variant number determining part 42 to factorize the data matrix into the product of an intensity distribution matrix and a matrix representing a basis spectrum. The method for matrix factorization is as have already been described. Result thereof is transferred to a second NMF processing part 44.

The second NMF processing part 44 is a function realized by causing the processor 33 to execute the program stored in the storage device 34. The second NMF processing part 44 performs non-negative matrix factorization on the intensity distribution matrix provided from the first NMF processing part 43 to factorize the matrix into the product of a matrix representing the mass proportion of a model polymer formed only from a polyad in a sample, and a matrix representing a feature vector of the model polymer. The method for matrix factorization is as have already been described. Result thereof is transferred to a vector projection part 45.

The vector projection part 45 is a function realized by causing the processor 33 to execute the program stored in the storage device 34. The vector projection part 45 defines the feature vector of the model polymer provided from the second NMF processing part 44 as an end member, and determines a K-1 dimensional simplex including all of the feature vectors of the samples. The method for determining the K-1 dimensional simplex is as have already been described.

The composition inference part 46 is a function realized by causing the processor 33 to execute the program stored in the storage device 34. The composition inference part 46 calculate a distance between each of the K end members in the K-1 dimensional simplex determined by the vector projection part 45 and the feature vector of the inference target sample, and infers each polyad content ratio of the inference target sample by the distance ratio. The composition inference part 46 outputs sequence analysis result through the input-output I/F 35 to the outside 48.

In the present embodiment, while the sequence analysis result is output through the input-output I/F 35 to the outside 48, the sequence analysis result may be transmitted to an external network using communication in addition to the input-output I/F 35.

According to the sequence analyzing device 30, even for a polymer which is not easy to prepare a reference sample, accurate sequence analysis can be made without particular preparatory process by a simple procedure. The sequence analyzing device 30 achieves significant reduction in time required for obtaining result, particularly when being applied to quality control of a polymer synthesized from a plurality of monomers, investigation of a cause for failure, etc.

[Sequence analyzing device (second embodiment)]

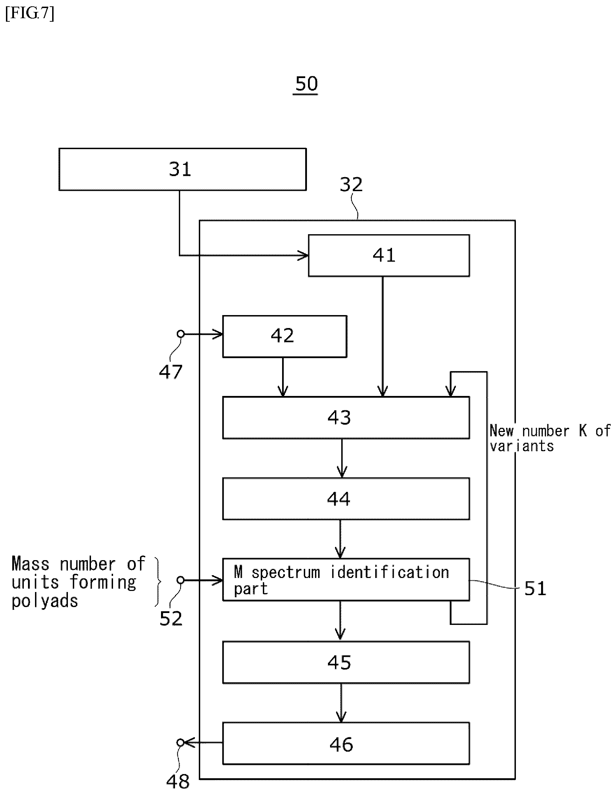

Fig. 7 is a functional block diagram of a sequence analyzing device according to the second embodiment. The sequence analyzing device 50 is the same as the sequence analyzing device 30 except that the information processing device 32 has a model polymer spectrum identification part 51 (shown as "M spectrum identification part" in the diagram). A difference from the sequence analyzing device 30 will be described below.

The model polymer spectrum identification part 51 of the sequence analyzing device 50 is a function realized by causing the processor 33 to execute the program stored in the storage device 34. The model polymer spectrum identification part 51 identifies a polyad to which an M spectrum is attributed by the basis spectra provided from the first NMF processing part 43, the feature vectors of the model polymers provided from the second NMF processing part 44, and mass numbers of units forming the polyads acquired from the outside 52. The details of the identification method are as have already been described. The mass numbers of units forming the polyads may be previously stored in the storage device 34.

When an M spectrum which is not attributed to any polyad is present, the model polymer spectrum identification part 51 provides a new number K of variants (typically, K is a number obtained by subtracting a predetermined number from the original number K of variants) to the first NMF processing part 43, and makes the first NMF processing part 43 and the second NMF processing part 44 to perform first NMF process and second NMF process, respectively. These processes are repeatedly performed until every M spectrum can be attributed to each of polyads. The method for reducing the number of variants is not particularly limited, and a method by subtracting 1 is typically used.

When all the M spectra are attributed to polyads by the M spectrum identification part 51, a matrix representing the mass proportion of a model polymer calculated by the second NMF processing part 44 in a sample and a matrix representing a feature vector of the model polymer are provided to the vector projection part 45. The subsequent process is the same as for the sequence analyzing device 30.

The sequence analyzing device 50 has the model polymer spectrum identification part 51. Therefore, even if K originally determined as a value which can be theoretically taken does not actually conform to the value and the reason is unknown, a proper K is reset by predetermined processes, and more accurate sequence analysis result can be provided.

[Polymerization condition proposal device]

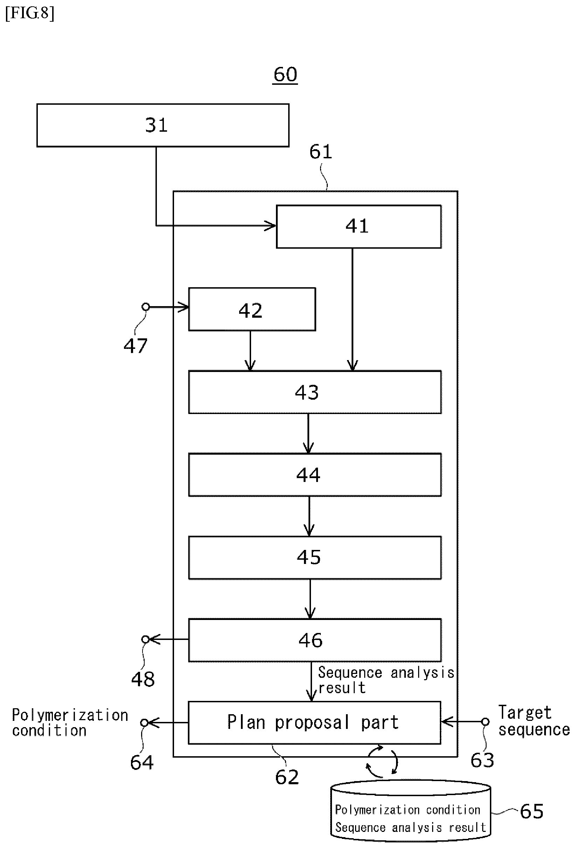

A polymerization condition proposal device according to an embodiment of the present invention will be described below with reference to drawings. Fig. 8 is a functional block diagram of the polymerization condition proposal device according to one embodiment of the present invention.

The polymerization condition proposal device 60 has an information processing device 61. The information processing device 61 has further a plan proposal part 62 in addition to the function of the sequence analyzing device 30. The hardware of the polymerization condition proposal device 60 is the same as of the sequence analyzing device 30, and the plan proposal part 62 is a function realized by causing the processor 33 to execute the program stored in the storage device 34. The function of the polymerization condition proposal device 60 is the same as of the sequence analyzing device 30 except for having the plan proposal part 62, and thus the function of the plan proposal part 62 will be described below.

The plan proposal part 62 is a learning model created by machine learning which uses as training data a plurality of actual measurement data in which a polymerization condition, and sequence analysis result of a polymer obtained as a result are associated with each other.

The plan proposal part 62 generates a polymerization condition data set including a plurality of polymerization conditions for polymers obtained having unknown sequences, and calculates prediction result (polymer sequence) for each of the polymerization conditions.

The plan proposal part 62 further generates a prediction data set in which a polymerization condition and the prediction result are associated with each other, identifies prediction result close to a target sequence of the obtained prediction result, extracts a polymerization condition which is associated with the identified prediction result.

The plan proposal part 62 receives data in which sequence analysis result of the inference target sample and the polymerization condition thereof are associated with each other from the composition inference part 46, and further receives target sequence data through the input-output I/F 35 from the outside 63.

The plan proposal part 62 generates a plurality of polymerization condition data sets, and predicts a sequence. Particularly, a condition to obtain a sequence closer to the target sequence than a sequence from sequence analysis result acquired from the composition inference part 46 is extracted, and this is defined as the "polymerization condition" and proposed through the input-output I/F 35 to the outside 64.

The learning model may be, for example, a learned neural network which has been learned using each parameter of polymerization conditions as an explanation variable and sequence analysis result of the obtained polymer as an objective variable. Known methods for building such learning model can be used, and methods described in WO2020/054183, WO2020/066309 and Japanese Translation of PCT International Application Publication No. 2008-501837, for example, can be used.

The polymerization condition proposal device 60 has the plan proposal part 62, and thus can propose a polymerization condition, which is expected to obtain a polymer having a sequence closer to the target sequence by comparing sequence analysis result and the target sequence. According to the foregoing, even in a complicated system having a larger number of types of monomers and/or longer polyads, materials can be more efficiently designed.

While the polymerization condition proposal device 60 does not have the model polymer spectrum identification part 51, the polymerization condition proposal device of the present invention preferably has the model polymer spectrum identification part 51.

[Automatic synthesizing device]

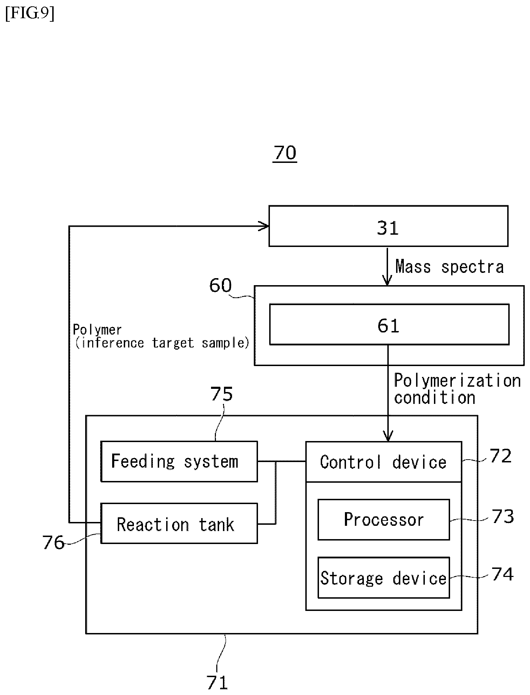

An automatic synthesizing device according to an embodiment of the present invention will be described below with reference to drawings. Fig. 9 is a functional block diagram of an automatic synthesizing device according to one embodiment of the present invention.

The automatic synthesizing device 70 further has a polymer synthesizing device 71 in addition to the polymerization condition proposal device 60.

The polymer synthesizing device 71 includes a monomer feeding system 75, a reaction tank 76, and a control device 72 that controls the above.

While a part of the function of the polymerization condition proposal device 60 is omitted and only portions necessary for description are shown in Fig. 8, the device has the same function as of the polymerization condition proposal device 60 described above.

The polymer synthesizing device 71 may be typically a flow reactor. The monomer feeding system 75 feeds monomers and/or a monomer solution having monomers solved in a solvent to the reaction tank 76.

The synthesizing device 71 may have a plurality of monomer feeding systems 75, and these are each individually controlled by the control device 72. The monomer feeding system 75 typically has a container that retains monomers (or a solution), a pipeline from the container to the reaction tank 76, and a pump. The types and amount of monomers fed to the reaction tank 76 are adjusted by the power of the pump.

The reaction tank 76 is a hollow portion provided in the pipeline connected to the feeding system 75, and is typically a container-shaped reaction site. The reaction tank 76 has e.g. a heater, a gas pipeline for atmosphere adjustment, a valve, a pump and stirring wings.

The monomer feeding system 75 and the reaction tank 76 are controlled by causing the processor 73 to execute the program stored in the storage device 74. Particularly, by receiving a polymerization condition provided from the polymerization condition proposal device 60, specifically a polymerization condition which is predicted to obtain a polymer having a sequence closer to the target sequence, the feeding system 75 is controlled along the condition, and the types of monomers fed to the reaction tank 76 and the amount of each monomer fed are adjusted. The reaction tank 76 is also controlled, and the reaction temperature, reaction time, and stirring rate, for example, are adjusted.

After completion of the reaction for a predetermined time, the control device 72 controls the pump of the reaction tank 76 to transfer the obtained polymer from the reaction tank 76 to the mass spectrometer 31.

In the automatic synthesizing device 70, the reaction tank 76 and the mass spectrometer 31 are connected by the pipeline, and the synthesized polymer is subjected to sequence analysis again.

According to the automatic synthesizing device 70 configured as described above, polymerization is automatically performed in a polymerization condition proposed from the polymerization condition proposal device 60, the obtained polymerized substance is further subjected to sequence analysis again, and the result is evaluated repeatedly. Thus, a polymer is automatically synthesized along the target sequence.

Examples

The present invention will now be described by way of Examples thereof. It should be noted, however, that the present invention is not limited thereto.

[Example 1: Analysis of MMA/St/BA triad]

Triad analysis was performed using methyl methacrylate (M), styrene (S) and butyl acrylate as a monomer set.

Methyl methacrylate, styrene and butyl acrylate, each available from Tokyo Chemical Industry Co., Ltd., were used.

These monomers were injected into a vial in predetermined amounts, and dimethyl 2,2'-azobis(isobutyrate) was added thereto as a polymerization initiator. After nitrogen gas replacement, polymerization was performed with stirring at a predetermined temperature for a predetermined time, and the reaction was then stopped by methanol. The obtained polymer was dried and then subjected to mass spectrometry by "DART-MS."

The procedure of mass spectrometry by "DART-MS" is as follows. The polymer was heated at a temperature increasing rate of 50°C/min from 50°C to 500°C on a heater (product name "ionRocket" available from Biochromato, Inc.) and pyrolyzed. Measurement was performed for 11 minutes including a preheating time of 2 minutes from room temperature to 50°C per sample. Pyrolysis gas was sequentially ionized by excited He gas using "DART"-ion source (product name "DART-OS" available from IonSense, Inc.)

The spectra of MS ("LCMS-2020" available from Shimadzu Corporation) were recorded at 50 scan/min in a positive ion mode, and 550 spectra were obtained per sample. The mass range was 50-1500 m/z, the interval scale was 0.05 m/z, and the mass resolution was 2000.

The spectra were output in CDF file format, and converted to Numpy format with netCDF4, a Python module. All the data processing were performed on Python 3.7 on a Windows 11 laptop computer with AMD Ryzen9 4900HS without external GPU assistance. The total process time was 2-3 hours.

The tables below summarize the polymerization conditions of M/S/B three-component systems. In the tables, "mass (mg)" represents the mass of the obtained polymer, "M initial fraction," "S initial fraction" and "B initial fraction" represent the added ratio (in terms of mass) of M, S and B, respectively, and "polym. Time (h)" and "polym. Temp (C)" represent reaction time (h) and reaction temperature (°C), respectively. As shown in the tables below, 85 types of polymers different from each other were synthesized in different reaction conditions.

[Table 4]

| Sample No. | 1 | 2 | 3 | 4 | 5 | 6 | 7 | 8 | 9 | 10 |
|---|---|---|---|---|---|---|---|---|---|---|
| mass (mg) | 0.34 | 0.36 | 0.41 | 0.29 | 0.37 | 0.41 | 0.36 | 0.39 | 0.36 | 0.34 |
| M initial fraction | 0 | 0.05 | 0.1 | 0.1 | 0.1 | 0.15 | 0.15 | 0.15 | 0.2 | 0.2 |
| S initial fraction | 0 | 0.95 | 0 | 0.9 | 0.9 | 0 | 0.85 | 0.85 | 0 | 0.8 |
| B initial fraction | 1 | 0 | 0.9 | 0 | 0 | 0.85 | 0 | 0 | 0.8 | 0 |
| polym. Time (h) | 1 | 1 | 5 | 1 | 1 | 1 | 1 | 1 | 1 | 1 |
| polym. Temp. (C) | 60 | 60 | 60 | 40 | 60 | 60 | 60 | 60 | 60 | 60 |
| Sample No. | 11 | 12 | 13 | 14 | 15 | 16 | 17 | 18 | 19 | 20 |
| mass (mg) | 0.34 | 0.22 | 0.38 | 0.42 | 0.36 | 0.39 | 0.28 | 0.36 | 0.4 | 0.4 |
| M initial fraction | 0.2 | 0.25 | 0.3 | 0.3 | 0.3 | 0.3 | 0.3 | 0.3 | 0.33 | 0.35 |
| S initial fraction | 0.8 | 0.25 | 0 | 0 | 0 | 0.4 | 0.7 | 0.7 | 0.33 | 0 |
| B initial fraction | 0 | 0.5 | 0.7 | 0.7 | 0.7 | 0.3 | 0 | 0 | 0.33 | 0.65 |
| polym. Time (h) | 1 | 3 | 1 | 5 | 3 | 2 | 1 | 1 | 2 | 1 |
| polym. Temp. (C) | 60 | 40 | 60 | 60 | 40 | 60 | 40 | 60 | 60 | 60 |
| Sample No. | 21 | 22 | 23 | 24 | 25 | 26 | 27 | 28 | 29 | 30 |
| mass (mg) | 0.35 | 0.42 | 0.46 | 0.43 | 0.3 | 0.42 | 0.38 | 0.45 | 0.37 | 0.41 |
| M initial fraction | 0.35 | 0.35 | 0.4 | 0.4 | 0.4 | 0.4 | 0.4 | 0.4 | 0.4 | 0.45 |
| S initial fraction | 0.65 | 0.65 | 0 | 0 | 0 | 0.4 | 0.6 | 0.6 | 0.6 | 0 |
| B initial fraction | 0 | 0 | 0.6 | 0.6 | 0.6 | 0.2 | 0 | 0 | 0 | 0.55 |
| polym. Time (h) | 1 | 1 | 1 | 5 | 3 | 1 | 1 | 1 | 1 | 1 |
| polym. Temp. (C) | 40 | 60 | 60 | 60 | 40 | 60 | 40 | 60 | 60 | 60 |
| Sample No. | 31 | 32 | 33 | 34 | 35 | 36 | 37 | 38 | 39 | 40 |
| mass (mg) | 0.37 | 0.34 | 0.5 | 0.44 | 0.28 | 0.36 | 0.37 | 0.39 | 0.39 | 0.36 |
| M initial fraction | 0.45 | 0.45 | 0.5 | 0.5 | 0.5 | 0.5 | 0.5 | 0.55 | 0.55 | 0.6 |
| S initial fraction | 0 | 0.55 | 0 | 0 | 0 | 0.5 | 0.5 | 0 | 0.45 | 0 |
| B initial fraction | 0.55 | 0 | 0.5 | 0.5 | 0.5 | 0 | 0 | 0.45 | 0 | 0.4 |
| polym. Time (h) | 5 | 1 | 1 | 5 | 3 | 1 | 1 | 1 | 1 | 1 |
| polym. Temp. (C) | 60 | 60 | 60 | 60 | 40 | 40 | 60 | 60 | 60 | 60 |
| Sample No. | 41 | 42 | 43 | 44 | 45 | 46 | 47 | 48 | 49 | 50 |
| mass (mg) | 0.37 | 0.36 | 0.41 | 0.39 | 0.38 | 0.37 | 0.37 | 0.39 | 0.46 | 0.36 |
| M initial fraction | 0.6 | 0.6 | 0.6 | 0.65 | 0.65 | 0.7 | 0.7 | 0.7 | 0.7 | 0.75 |
| S initial fraction | 0 | 0.4 | 0.4 | 0 | 0.35 | 0 | 0 | 0.3 | 0.3 | 0 |
| B initial fraction | 0.4 | 0 | 0 | 0.35 | 0 | 0.3 | 0.3 | 0 | 0 | 0.25 |
| polym. Time (h) | 5 | 1 | 1 | 1 | 1 | 1 | 1 | 1 | 1 | 1 |

(continued)

| Sample No. | 41 | 42 | 43 | 44 | 45 | 46 | 47 | 48 | 49 | 50 |
|---|---|---|---|---|---|---|---|---|---|---|
| polym. Temp. (C) | 60 | 40 | 60 | 60 | 60 | 60 | 60 | 40 | 60 | 60 |

[Table 5]

| Sample No. | 51 | 52 | 53 | 54 | 55 | 56 | 57 | 58 | 59 | 60 |
|---|---|---|---|---|---|---|---|---|---|---|
| mass (mg) | 0.34 | 0.39 | 0.41 | 0.35 | 0.37 | 0.4 | 0.4 | 0.35 | 0.32 | 0.38 |
| M initial fraction | 0.8 | 0.8 | 0.8 | 0.8 | 0.85 | 0.85 | 0.85 | 0.9 | 0.9 | 0.9 |
| S initial fraction | 0 | 0 | 0.2 | 0.2 | 0 | 0 | 0.15 | 0 | 0 | 0.1 |
| B initial fraction | 0.2 | 0.2 | 0 | 0 | 0.15 | 0.15 | 0 | 0.1 | 0.1 | 0 |
| polym. Time (h) | 1 | 1 | 1 | 1 | 1 | 1 | 1 | 1 | 1 | 1 |
| polym. Temp. (C) | 60 | 60 | 60 | 60 | 60 | 60 | 60 | 60 | 60 | 60 |
| Sample No. | 61 | 62 | 63 | 64 | 65 | 66 | 67 | 68 | 69 | 70 |
| mass (mg) | 0.3 | 0.37 | 0.38 | 0.36 | 0.45 | 0.43 | 0.47 | 0.48 | 0.37 | 0.42 |
| M initial fraction | 0.9 | 0.95 | 0.95 | 0.95 | 1 | 0 | 0 | 0 | 0 | 0 |
| S initial fraction | 0.1 | 0 | 0 | 0.05 | 0 | 0.1 | 0.15 | 0.25 | 0.3 | 0.4 |
| B initial fraction | 0 | 0.05 | 0.05 | 0 | 0 | 0.9 | 0.85 | 0.75 | 0.7 | 0.6 |
| polym. Time (h) | 1 | 1 | 1 | 1 | 1 | 3 | 1 | 1 | 1 | 1 |
| polym. Temp. (C) | 60 | 60 | 60 | 60 | 60 | 60 | 60 | 60 | 60 | 60 |
| Sample No. | 71 | 72 | 73 | 74 | 75 | 76 | 77 | 78 | 79 | 80 |
| mass (mg) | 0.46 | 0.52 | 0.4 | 0.43 | 0.28 | 0.4 | 0.26 | 0.36 | 0.4 | 0.36 |
| M initial fraction | 0 | 0 | 0 | 0 | 0 | 0 | 0 | 0 | 0 | 0 |
| S initial fraction | 0.4 | 0.45 | 0.45 | 0.5 | 0.5 | 0.55 | 0.6 | 0.6 | 0.65 | 0.7 |
| B initial fraction | 0.6 | 0.55 | 0.55 | 0.5 | 0.5 | 0.45 | 0.4 | 0.4 | 0.35 | 0.3 |
| polym. Time (h) | 1 | 1 | 1 | 1 | 1 | 1 | 1 | 1 | 1 | 1 |
| polym. Temp. (C) | 40 | 40 | 60 | 60 | 40 | 60 | 40 | 60 | 60 | 60 |
| Sample No. | 81 | 82 | 83 | 84 | 85 | | | | | |
| mass (mg) | 0.37 | 0.4 | 0.38 | 0.33 | 0.37 | | | | | |
| M initial fraction | 0 | 0 | 0 | 0 | 1 | | | | | |
| S initial fraction | 0.75 | 0.8 | 0.9 | 1 | 1 | | | | | |
| B initial fraction | 0.25 | 0.2 | 0.1 | 0 | 0 | | | | | |
| polym. Time (h) | 1 | 1 | 1 | 1 | 1 | | | | | |
| polym. Temp. (C) | 60 | 60 | 60 | 60 | 60 | | | | | |

As described above, the number of samples was 85, the temperature range for mass spectrometry was 200-450°C, and the m/z range was 50-410. Other hyperparameters are as follows.

[Table 6]

| First NMF | | | | | Second NMF | | |
|---|---|---|---|---|---|---|---|
| $w_o$ | Merging threshold | Initial $M$ | Prior | iteration | $K$ | $\alpha = \beta$ | $p$ |
| 0.2 | 0.99 | 60 | Exp | 10000 | 13 | 0.05 | 1 |

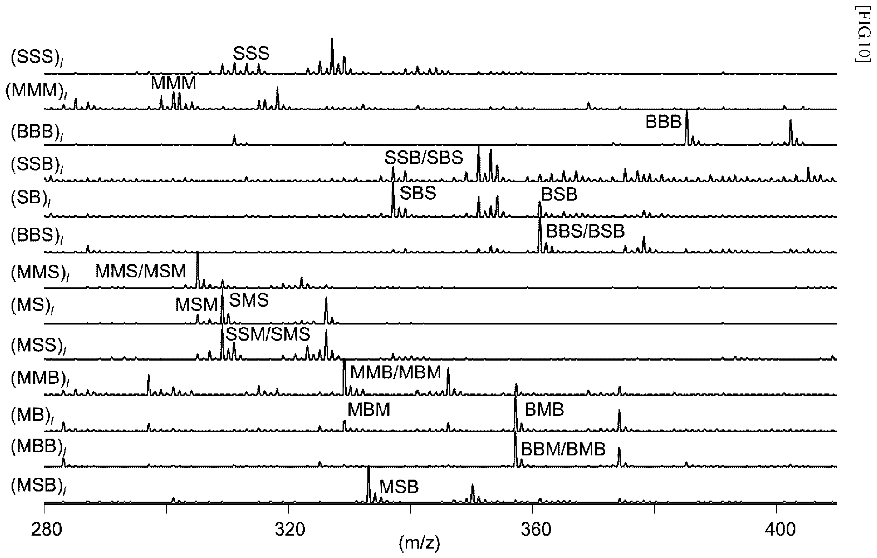

Fig. 10 shows model polymer spectra of polyads obtained by calculation. The number of types of monomers was 3, and for triad analysis, K was 13. Every spectrum had peaks at reasonable positions when making comparisons with the sum of mass of the monomers. Additionally, "(XXX)$_1$" described at the side of each spectrum in Fig. 10 represents the type of polyads, and "XXX" described at a peak position represents a peak position of the identified polyad.

Each model polymer spectrum in Fig. 10 was reasonably attributed to each triad, and it was revealed that calculation could be performed as intended.

[Example 2: Analysis of St/BA pentad]

Sequences were analyzed in the same manner as in Example 1 except that the monomer set was changed from MMA/St/BA to St/BA and pentads were analyzed. Tables below are the tables of polymerization conditions. In the tables, "mass (mg)" represents the mass of the obtained polymer, "S initial fraction" and "B initial fraction" represent the added ratio (in terms of mass) of S and B, respectively, and "polym. Time (h)" and "polym. Temp (C)" represent reaction time (h) and reaction temperature (°C), respectively. As shown in the tables below, 81 types of polymers different from each other were synthesized in different reaction conditions.

[Table 7]

| Sample No. | 1 | 2 | 3 | 4 | 5 | 6 | 7 | 8 | 9 | 10 |
|---|---|---|---|---|---|---|---|---|---|---|
| mass (mg) | 0.34 | 0.37 | 0.47 | 0.43 | 0.51 | 0.47 | 0.47 | 0.41 | 0.4 | 0.34 |
| S initial fraction | 0 | 0.05 | 0.1 | 0.1 | 0.1 | 0.15 | 0.15 | 0.15 | 0.15 | 0.15 |
| B initial fraction | 1 | 0.95 | 0.9 | 0.9 | 0.9 | 0.85 | 0.85 | 0.85 | 0.85 | 0.85 |
| polym. Time (h) | 1 | 1 | 1 | 3 | 0.25 | 1 | 1 | 1 | 0.08 | 1 |
| polym. Temp. (C) | 60 | 60 | 60 | 60 | 150 | 60 | 40 | 40 | 150 | 150 |
| Sample No. | 11 | 12 | 13 | 14 | 15 | 16 | 17 | 18 | 19 | 20 |
| mass (mg) | 0.57 | 0.5 | 0.28 | 0.48 | 0.49 | 0.39 | 0.4 | 0.43 | 0.37 | 0.54 |
| S initial fraction | 0.2 | 0.2 | 0.2 | 0.25 | 0.25 | 0.25 | 0.25 | 0.25 | 0.3 | 0.3 |
| B initial fraction | 0.8 | 0.8 | 0.8 | 0.75 | 0.75 | 0.75 | 0.75 | 0.75 | 0.7 | 0.7 |
| polym. Time (h) | 1 | 0.08 | 1 | 1 | 1 | 1 | 0.08 | 1 | 1 | 3 |
| polym. Temp. (C) | 40 | 150 | 150 | 60 | 40 | 40 | 150 | 150 | 60 | 60 |
| Sample No. | 21 | 22 | 23 | 24 | 25 | 26 | 27 | 28 | 29 | 30 |
| mass (mg) | 0.3 | 0.4 | 0.5 | 0.38 | 0.47 | 0.65 | 0.37 | 0.38 | 0.35 | 0.42 |
| S initial fraction | 0.3 | 0.3 | 0.3 | 0.3 | 0.3 | 0.3 | 0.3 | 0.35 | 0.35 | 0.35 |
| B initial fraction | 0.7 | 0.7 | 0.7 | 0.7 | 0.7 | 0.7 | 0.7 | 0.65 | 0.65 | 0.65 |
| polym. Time (h) | 5 | 1 | 15 | 1 | 0.08 | 1 | 1 | 1 | 1 | 1 |
| polym. Temp. (C) | 60 | 40 | 40 | 150 | 150 | 150 | 60 | 40 | 40 | 150 |
| Sample No. | 31 | 32 | 33 | 34 | 35 | 36 | 37 | 38 | 39 | 40 |
| mass (mg) | 0.42 | 0.43 | 0.28 | 0.46 | 0.28 | 0.43 | 0.52 | 0.42 | 0.4 | 0.43 |
| S initial fraction | 0.4 | 0.4 | 0.4 | 0.4 | 0.4 | 0.4 | 0.45 | 0.45 | 0.45 | 0.5 |
| B initial fraction | 0.6 | 0.6 | 0.6 | 0.6 | 0.6 | 0.6 | 0.55 | 0.55 | 0.55 | 0.5 |
| polym. Time (h) | 1 | 3 | 1 | 1 | 15 | 1 | 1 | 1 | 1 | 1 |
| polym. Temp. (C) | 60 | 60 | 40 | 40 | 40 | 60 | 40 | 60 | 60 | 60 |
| Sample No. | 41 | 42 | 43 | 44 | 45 | 46 | 47 | 48 | 49 | 50 |
| mass (mg) | 0.43 | 0.28 | 0.4 | 0.52 | 0.48 | 0.23 | 0.36 | 0.39 | 0.4 | 0.36 |
| S initial fraction | 0.5 | 0.5 | 0.5 | 0.5 | 0.5 | 0.55 | 0.55 | 0.55 | 0.55 | 0.55 |
| B initial fraction | 0.5 | 0.5 | 0.5 | 0.5 | 0.5 | 0.45 | 0.45 | 0.45 | 0.45 | 0.45 |
| polym. Time (h) | 3 | 1 | 1 | 15 | 0.08 | 1 | 15 | 0.08 | 1 | 1 |

(continued)

| Sample No. | 41 | 42 | 43 | 44 | 45 | 46 | 47 | 48 | 49 | 50 |
|---|---|---|---|---|---|---|---|---|---|---|
| polym. Temp. (C) | 60 | 40 | 40 | 40 | 150 | 40 | 40 | 150 | 150 | 60 |

[Table 8]

| Sample No. | 51 | 52 | 53 | 54 | 55 | 56 | 57 | 58 | 59 | 60 |
|---|---|---|---|---|---|---|---|---|---|---|
| mass (mg) | 0.4 | 0.2 | 0.26 | 0.38 | 0.39 | 0.37 | 0.44 | 0.26 | 0.42 | 0.34 |
| S initial fraction | 0.55 | 0.6 | 0.6 | 0.6 | 0.6 | 0.6 | 0.65 | 0.65 | 0.65 | 0.65 |
| B initial fraction | 0.45 | 0.4 | 0.4 | 0.4 | 0.4 | 0.4 | 0.35 | 0.35 | 0.35 | 0.35 |
| polym. Time (h) | 1 | 1 | 15 | 1 | 0.08 | 1 | 1 | 1 | 1 | 1 |
| polym. Temp. (C) | 60 | 40 | 40 | 150 | 150 | 150 | 40 | 150 | 150 | 60 |
| Sample No. | 61 | 62 | 63 | 64 | 65 | 66 | 67 | 68 | 69 | 70 |
| mass (mg) | 0.4 | 0.36 | 0.38 | 0.38 | 0.33 | 0.5 | 0.37 | 0.32 | 0.32 | 0.4 |
| S initial fraction | 0.65 | 0.7 | 0.7 | 0.7 | 0.7 | 0.7 | 0.75 | 0.75 | 0.75 | 0.8 |
| B initial fraction | 0.35 | 0.3 | 0.3 | 0.3 | 0.3 | 0.3 | 0.25 | 0.25 | 0.25 | 0.2 |
| polym. Time (h) | 1 | 1 | 3 | 5 | 1 | 1 | 1 | 1 | 1 | 1 |
| polym. Temp. (C) | 60 | 60 | 60 | 60 | 150 | 60 | 60 | 40 | 150 | 60 |
| Sample No. | 71 | 72 | 73 | 74 | 75 | 76 | 77 | 78 | 79 | 80 |
| mass (mg) | 0.4 | 0.36 | 0.36 | 0.36 | 0.34 | 0.31 | 0.39 | 0.38 | 0.39 | 0.45 |
| S initial fraction | 0.8 | 0.8 | 0.8 | 0.8 | 0.85 | 0.85 | 0.85 | 0.9 | 0.9 | 0.95 |
| B initial fraction | 0.2 | 0.2 | 0.2 | 0.2 | 0.15 | 0.15 | 0.15 | 0.1 | 0.1 | 0.05 |
| polym. Time (h) | 3 | 5 | 1 | 1 | 1 | 1 | 3 | 1 | 3 | 1 |
| polym. Temp. (C) | 60 | 60 | 40 | 60 | 60 | 60 | 60 | 60 | 60 | 60 |
| Sample No. | 81 | | | | | | | | | |
| mass (mg) | 0.33 | | | | | | | | | |
| S initial fraction | 1 | | | | | | | | | |
| B initial fraction | 0 | | | | | | | | | |
| polym. Time (h) | 1 | | | | | | | | | |
| polym. Temp. (C) | 60 | | | | | | | | | |

As described above, the number of samples was 81, the temperature range for mass spectrometry was 200-450°C, and the m/z range was 100-700. Other hyperparameters are as follows.

[Table 9]

| First NMF | | | | | Second NMF | | |
|---|---|---|---|---|---|---|---|
| $w_o$ | Merging threshold | Initial $M$ | Prior | iteration | $K$ | $\alpha = \beta$ | $p$ |
| 0.2 | 0.99 | 60 | Exp | 10000 | 9 | 0.01 | 1 |

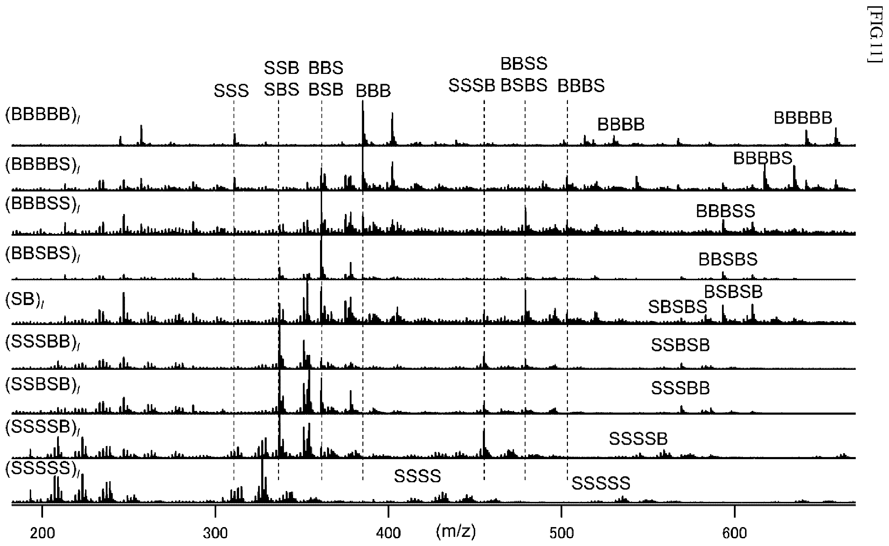

Analysis was performed as K = 9. When the number of types of monomers is 2, the number of theoretical combinations is 9 when the length of polyads is 5. Fig. 11 is result of the model polymer spectra.

Every spectrum had peaks at reasonable positions when making comparisons with the sum of mass of the monomers. Additionally, "$(XXXXX)_1$" described at the side of each spectrum in Fig. 11 represents the type of polyads, and "XXXXX" described at a peak position represents a peak position of the identified polyad.

Each model polymer spectrum in Fig. 11 was reasonably attributed to each triad, and it was revealed that calculation could be performed as intended.

[Example 3: Comparison with NMR measurement result]

Polymers were grown in a living radical polymerization system using two types of monomers, styrene (S) and butyl acrylate (B), and samples obtained by sampling over time were analyzed using the basis spectra of the pentads learned in Example 2. The same samples were further analyzed by NMR and also compared with theoretical curves calculated by the Alfrey-Mayo equation.

Only the information of "B"-centered triads is obtained by NMR, and thus basis spectra of the triad composition were newly created by downgrading (integrating) the basis spectra of the pentads learned in Example 2 for comparison with the NMR data. The method will be described below in detail.

First, the pentad composition of the inference target sample is obtained by projection onto a hyperplane spanned by S and B along the method which has already been described. This is defined as $C_{test}$ (here, K-9). The following is multiplied by transformation matrix from pentad to triad from the right side:

[Formula 166]

$$C_{test} \in \mathbb{R}_+^{1 \times K}$$

.

The transformation matrix is as shown in a table below.

[Table 10]

| Sequence-defined copolymers | B-centered triad matrix, $T_B$ | | | S-centered triad matrix, $T_S$ | | |
|---|---|---|---|---|---|---|
| | BBB | BBS | SBS | SSS | SSB | BSB |
| $(BBBBB)_l$ | 5 | 0 | 0 | 0 | 0 | 0 |
| $(BBBBS)_l$ | 2 | 2 | 0 | 0 | 0 | 1 |
| $(BBBSS)_l$ | 1 | 2 | 0 | 0 | 2 | 0 |
| $(BBSBS)_l$ | 0 | 2 | 1 | 0 | 0 | 2 |
| $(BS)_l$ | 0 | 0 | 2.5 | 0 | 0 | 2.5 |
| $(SSBSB)_l$ | 0 | 0 | 2 | 0 | 2 | 1 |
| $(SSSBB)_l$ | 0 | 2 | 0 | 1 | 2 | 0 |
| $(SSSSB)_l$ | 0 | 0 | 1 | 2 | 2 | 0 |
| $(SSSSS)_l$ | 0 | 0 | 0 | 5 | 0 | 0 |

In the table above, "Sequence-defined copolymers" is a matrix representing a basis spectrum of a pentad calculated in Example 2, "B-centered triad matrix, $T_B$" represents a transformation matrix to a B-centered (e.g. BBS, etc.) triad, and similarly "B-centered triad matrix, $T_S$" represents a transformation matrix to an S-centered triad. B-centered $T_B$, the data of which can be acquired by NMR, was used in this verification.

According to the above transformation matrix,

[Formula 167]

$$C_{test} T_{\mathrm{B}} \in \mathbb{R}_+^{1 \times 3}$$

the three-dimensional vector shown above is factorized into the mass ratio of BBB, BBS and SBS.

Living radical polymerization using two types of monomers, styrene (S) and butyl acrylate (B), was performed by the following procedure.

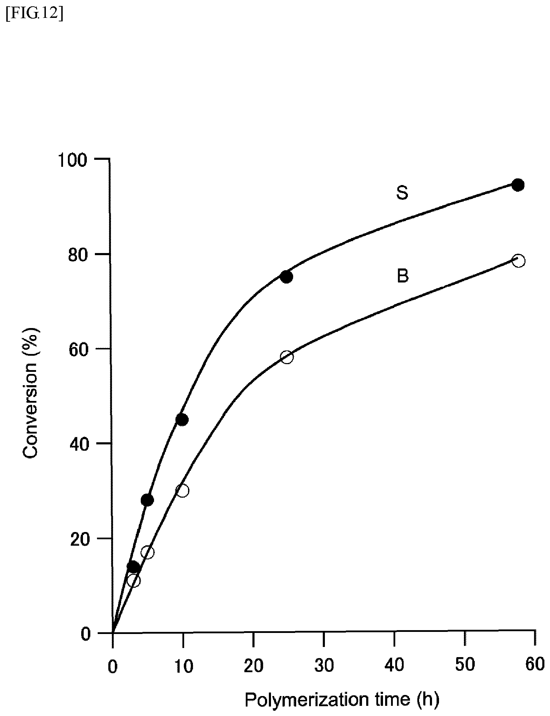

In a reaction container, 72.9 mg of 2-(dodecylthiocarbonothioylthio)-2-methylpropionic acid (DDMAT) and 9.9 mg of azobis(isobutyronitrile) (AIBN) were taken, and the atmosphere was replaced with nitrogen. In another container, 2.1 mL of styrene, 2 mL of n-butyl acrylate, and 2 mL of 1,4-dioxane were put, oxygen was removed by bubbling nitrogen gas for 30 minutes, and the mixture was added to the reaction container. The reaction container was heated to 70°C with stirring, and the obtained polymerization solution was sampled sometimes to analyze the conversion rate and sequence thereof. Fig. 12 is a graph showing a relationship between polymerization time and conversion rate.

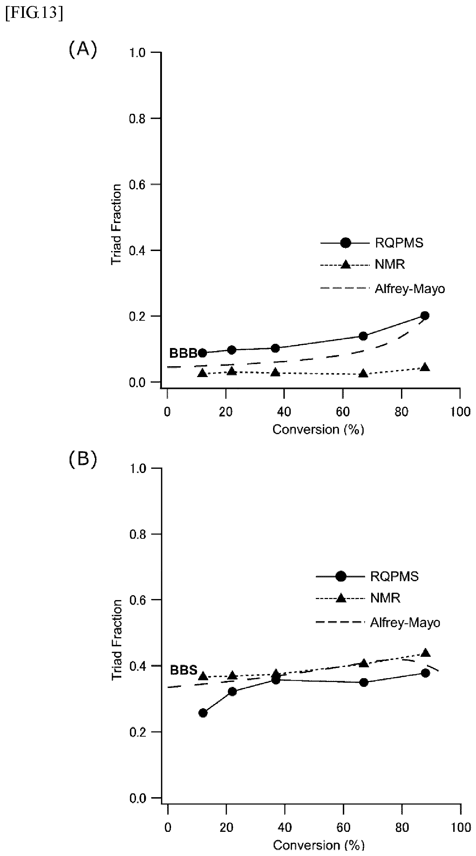

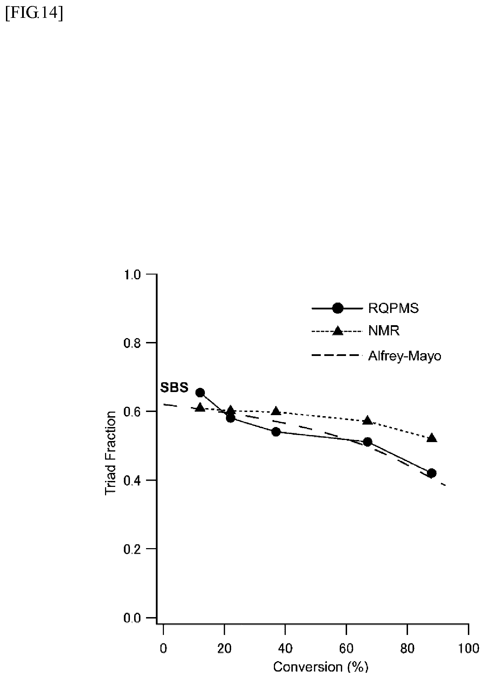

Figs. 13 and 14 are sequence analysis result. Fig. 13(A) shows changes in the BBB triad content of the obtained copolymer. The horizontal axis is conversion rate (%) and the vertical axis is the mass fraction of the BBB triad. Similarly, Fig. 13(B) shows changes in the BBS triad content of the obtained copolymer, and Fig. 14 shows changes in the SBS triad content.

In all triad analysis result, the NMR analysis result and the analysis result in Examples by the analysis method of the present invention "RQPMS; "reference-free" quantitative pyrolysis MS" agreed well with each other, and furthermore agreed with the theoretical curves calculated by the Alfrey-Mayo equation.

Reference Signs List

10, 20: K-1 dimensional simplex
12: Inscribed hypersphere
13, 14, 24, 25: End member
16, 17, 21-23: Reference sample
19: Region
30, 50: Sequence analyzing device
31: Mass spectrometer
32: Information processing device
33, 73: Processor
34, 74: Storage device
35: Input-output interface (I/F)
41: Data matrix generating part
42: Variant number determining part
43: First NMF processing part
44: Second NMF processing part
45: Vector projection part
46: Composition inference part
51: Model polymer spectrum identification part
60: Polymerization condition proposal device
62: Plan proposal part
70: Automatic synthesizing device
71: Synthesizing device
72: Control device
75: Feeding system
76: Reaction tank

**Claims**

1. A polymer sequence analysis method for inferring a polyad content of a polymer obtained by polymerizing monomers selected from a monomer set containing two or more types of monomers, which polyad is formed by aligning a plurality of units derived from the monomers, the method comprising,

    determining a number K of variants of the polyads depending on number of types of monomers contained in the monomer set, and number of the units forming the polyads,
    sequentially ionizing gas components generated by heating each sample of a reference sample and an inference target sample, which are polymers formed from the monomers, to acquire a data matrix comprising two-dimensional mass spectra having m/z with respect to heating temperature,
    performing first NMF process by which the data matrix is subjected to non-negative matrix factorization to be factorized into a product of a matrix representing a normalized basis spectrum and an intensity distribution matrix,
    performing second NMF process by which the intensity distribution matrix in each of the samples is subjected to non-negative matrix factorization to be factorized into a product of a matrix representing a mass proportion of a model polymer formed only from the polyad in the sample, and a matrix representing a feature vector of the model polymer,
    defining the feature vector of the model polymer as an end member and determining a K-1 dimensional simplex comprising all of the feature vectors of the samples, and
    defining a distance between the K end members and a feature vector of the inference target sample by Riemannian metric distance in consideration of non-orthogonality of a basis spectrum of first NMF process, and inferring each polyad content ratio of the inference target sample by a ratio of the distance.

2. The sequence analysis method according to claim 1, wherein when the number K of variants is equal to or greater than 3, at least one of the feature vectors of the reference samples is present in each region external to a hypersphere inscribed in the K-1 dimensional simplex or the reference samples contain at least one of the end members.

3. The sequence analysis method according to claim 1 or 2, further including,

    after the second NMF process,
    reconstructing a spectrum of the model polymer by a matrix product of a matrix representing the feature vector of the model polymer and a matrix representing the basis spectrum to perform an identification of the polyad to which the spectrum of the model polymer is attributed.

4. The sequence analysis method according to claim 3, wherein the identification is performed by comparing a sum of mass numbers of the units forming the polyad and m/z at peaks of the spectrum of the model polymer.

5. The sequence analysis method according to claim 3 or 4, wherein when a spectrum of the model polymer not attributed to any of the polyads is present as a result of the identification, a change in the number K of variants is made and the first NMF process, the second NMF process and the identification are repeated.

6. The sequence analysis method according to claim 5, wherein the change is to subtract a predetermined number from the number K of variants.

7. The sequence analysis method according to claim 3 or 4, wherein when a spectrum of the model polymer which cannot be attributed to the polyads is present as a result of the identification, the reference samples are added and the data matrix acquisition, the first NMF process, the second NMF process and the identification are repeated.

8. The sequence analysis method according to any one of claims 1 to 7, wherein when the number of types is j, and j is equal to or greater than 3, the number K of variants is determined by formula: $K = {}_jC_3 + 3{}_jC_2 + {}_jC_1$.

9. The sequence analysis method according to any one of claims 1 to 8, wherein the polymer comprises a resist resin.

10. The sequence analysis method according to any one of claims 1 to 9, wherein the reference sample and the inference target sample have different polymerization methods.

11. A polymer sequence analyzing device for inferring a polyad content of a polymer obtained by polymerizing monomers selected from a monomer set containing two or more types of monomers, which polyad is formed by aligning a plurality of units derived from the monomers, the device comprising,

a mass spectrometer that sequentially ionizes gas components generated by heating a sample comprising a reference sample and an inference target sample, which are polymers formed from the monomers, and observes mass spectra continuously, and
an information processing device that processes the observed mass spectra, wherein
the information processing device comprises,
a data matrix generating part that obtains a data matrix comprising two-dimensional mass spectra having m/z with respect to heating temperature,
a variant number determining part that determines number K of variants of the polyads depending on number of types of monomers contained in the monomer set and number of the units forming the polyads,
a first NMF processing part that performs NMF process by which the data matrix is subjected to non-negative matrix factorization to be factorized into a product of a matrix representing a normalized basis spectrum and an intensity distribution matrix,
a second NMF processing part that performs NMF process by which the intensity distribution matrix of each of the samples is subjected to non-negative matrix factorization to be factorized into a product of a matrix representing a mass proportion of a model polymer formed only from the polyad in the sample, and a matrix representing a feature vector of the model polymer and obtains the feature vector of the model polymer,
a vector projection part that defines the feature vector of the model polymer as an end member and determines a K-1 dimensional simplex comprising all of the feature vectors of the samples, and
a composition inference part that defines a distance between the K end members and a feature vector of the inference target sample by Riemannian metric distance in consideration of non-orthogonality of a basis spectrum of first NMF process, and infers each polyad content ratio of the inference target sample by a ratio of the distance.

12. The sequence analyzing device according to claim 11, further comprising a model polymer spectrum identification part that reconstructs a spectrum of the model polymer by a matrix product of a matrix representing the feature vector of the model polymer and a matrix representing the basis spectrum, and performs the identification of the polyad to which the spectrum of the model polymer is attributed.

13. The sequence analyzing device according to claim 12, wherein the identification is performed by comparing a sum of mass numbers of the units forming the polyad and m/z at peaks of the spectrum of the model polymer.

14. The sequence analyzing device according to claim 12 or 13, wherein when a spectrum of the model polymer not attributed to any of the polyads is present as a result of the identification, the information processing device changes the number K of variants and repeats NMF process by the first NMF processing part, NMF process by the second NMF processing part, and identification by the model polymer spectrum identification part.

15. A polymerization condition proposal device, further comprising,

the sequence analyzing device according to any one of claims 11 to 14, and
a plan proposal part that has been subjected to machine learning using sequence analysis result by the sequence analyzing device and polymerization conditions of the inference target sample as training data, wherein
the plan proposal part proposes a new polymerization condition to obtain, by comparing sequence analysis result and a predetermined target sequence, a polymer having the target sequence.

16. An automatic synthesizing device, having

the polymerization condition proposal device according to claim 15, and
a synthesizing device for the polymer, wherein
the synthesizing device has,
a feeding system for the monomers, a reaction tank that receives the monomers from the feeding system to allow the monomers to react, and a control device, and
the control device controls at least one selected from a group consisting of the feeding system and the reaction tank based on a polymerization condition proposed by the polymerization condition proposal device to synthesize a new polymer.

[FIG.1]

```
                    ┌─────────────────┐
                    │     START       │
                    └────────┬────────┘
                             │
                             ▼
            ┌──────────────────────────────┐
            │ Determining the number K of  │ ──── S1
            │ variants of polyads.         │
            └──────────────┬───────────────┘
                           │
                           ▼
            ┌──────────────────────────────┐
            │  Acquiring a data matrix.    │ ──── S2
            └──────────────┬───────────────┘
                           │
          ┌──────────────► │
          │                ▼
          │   ┌──────────────────────────────┐
          │   │     First NMF process.       │ ──── S3
          │   └──────────────┬───────────────┘
          │                  │
          │                  ▼
      YES │   ┌──────────────────────────────┐
          │   │    Second NMF process.       │ ──── S4
          │   └──────────────┬───────────────┘
          │                  │
          │                  ▼
          │   ┌──────────────────────────────┐
          │   │ Calculating model polymer    │ ──── S5
          │   │ spectra (M spectra).         │
          │   └──────────────┬───────────────┘
          │                  │
          │                  ▼
          │        Whether or not there is
          └──────  an M spectrum which is not  ──── S6
                   attributed to polyads.
                           │ NO
                           ▼
            ┌──────────────────────────────┐
            │  Determining a K-1           │ ──── S7
            │  dimensional simplex.        │
            └──────────────┬───────────────┘
                           │
                           ▼
            ┌──────────────────────────────┐
            │   Inferring polyad           │ ──── S8
            │   content ratios.            │
            └──────────────┬───────────────┘
                           │
                           ▼
                    ┌─────────────────┐
                    │      END        │
                    └─────────────────┘
```

[FIG.2]

EP 4 560 309 A1

EP 4 560 309 A1

[FIG.3]

59

[FIG.4]

[FIG.5]

30

| 31 | | 32 |
|---|---|---|

| 33 |
|---|

| 34 |
|---|

| 35 |
|---|

[FIG.6]

[FIG.7]

[FIG.8]

[FIG.9]

[FIG.10]

[FIG.11]

[FIG.12]

[FIG.13]

[FIG.14]

## INTERNATIONAL SEARCH REPORT

International application No.

**PCT/JP2023/017129**

| A. | CLASSIFICATION OF SUBJECT MATTER |
|---|---|

*G01N 27/62*(2021.01)i
FI:   G01N27/62 D

According to International Patent Classification (IPC) or to both national classification and IPC

| B. | FIELDS SEARCHED |
|---|---|

Minimum documentation searched (classification system followed by classification symbols)

G01N27/62; H01J49/00-49/48

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | US 2013/0035867 A1 (KATHOLIEKE UNIVERSITEIT LEUVEN K.U. LEUVEN R & D,) 07 February 2013 (2013-02-07) | 1-16 |
| A | JP 2018-512580 A (MICROMASS UK LTD.) 17 May 2018 (2018-05-17) | 1-16 |
| A | WO 2019/208225 A1 (SCREEN HOLDINGS CO., LTD.) 31 October 2019 (2019-10-31) | 1-16 |

☐ Further documents are listed in the continuation of Box C.          ☑ See patent family annex.

| | |
|---|---|
| *     Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **15 June 2023** | **27 June 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

International application No.

**PCT/JP2023/017129**

| Patent document cited in search report | | | Publication date (day/month/year) | Patent family member(s) | | | Publication date (day/month/year) |
|---|---|---|---|---|---|---|---|
| US | 2013/0035867 | A1 | 07 February 2013 | US | 2019/0236397 | A1 | |
| | | | | WO | 2011/127544 | A1 | |
| | | | | EP | 2558982 | A1 | |
| | | | | CA | 2795585 | A1 | |
| JP | 2018-512580 | A | 17 May 2018 | US | 2018/053644 | A1 | |
| | | | | EP | 3266037 | A1 | |
| | | | | KR | 10-2017-0130454 | A | |
| | | | | CA | 2978165 | A1 | |
| | | | | WO | 2016/142683 | A1 | |
| | | | | CN | 107530064 | A | |
| WO | 2019/208225 | A1 | 31 October 2019 | JP | 2021-165635 | A | |

Form PCT/ISA/210 (patent family annex) (January 2015)

**EP 4 560 309 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2019208225 A
- WO 2020054183 A
- WO 2020066309 A
- JP 2008501837 W

### Non-patent literature cited in the description

- **S. BOYD** ; **N. PARIKH** ; **E. CHU** ; **B. PELEATO** ; **J. ECKSTEIN**. Distributed optimization and statistical learning via the alternating direction method of multipliers. *Found. Trends Mach. Learn.*, 2010, vol. 3, 1-122
- ROBUST VOLUME MINIMIZATION-BASED MATRIX FACTORIZATION VIA ALTERNATING OPTIMIZATION. **X. FU** ; **W. MA** ; **K. HUANG** ; **N. D. SIDIROPOULOS**. Icassp 2016. University of Minnesota, 2016, 2534-2538
- **X. FU** ; **K. HUANG** ; **B. YANG** ; **W. K. MA** ; **N. D. SIDIROPOULOS**. Robust Volume Minimization-Based Matrix Factorization for Remote Sensing and Document Clustering. *IEEE Trans. Signal Process.*, 2016, vol. 64, 6254-6268
- **J. M. B. DIAS** ; **J. M. P. NASCIMENTO**. Vertex component analysis: A geometric-based approach to unmix hyperspectral data. *Signal Image Process. Remote Sens.*, 2006, vol. 43, 415-439

73